(19) Europäisches Patentamt
European Patent Office
Office européen des brevets

(11) **EP 4 488 386 A1**

(12) **EUROPEAN PATENT APPLICATION**

(43) Date of publication:
**08.01.2025 Bulletin 2025/02**

(21) Application number: **23306160.5**

(22) Date of filing: **07.07.2023**

(51) International Patent Classification (IPC):
**C12Q 1/6837** (2018.01)   **C12Q 1/6841** (2018.01)

(52) Cooperative Patent Classification (CPC):
(C-Sets available)
**C12Q 1/6837; C12Q 1/6841**   (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Scipio Bioscience**
**92120 Montrouge (FR)**

(72) Inventors:
• **FERNANDEZ, Nicolas**
**92120 Montrouge (FR)**
• **KOMATSU, Jun**
**92120 Montrouge (FR)**
• **ANDRE, Barbara**
**92120 Montrouge (FR)**

(74) Representative: **Icosa**
**83 avenue Denfert-Rochereau**
**75014 Paris (FR)**

(54) **COMPOSITIONS FOR SPATIAL OMICS**

(57)   The present invention relates to a composition comprising a population of capture barcode units and a population of positioning barcode units, and its use for determining the spatial location of any analyte from a biological sample.

**FIG. 3B**

EP 4 488 386 A1

(52) Cooperative Patent Classification (CPC): (Cont.)

C-Sets
**C12Q 1/6837, C12Q 2563/179, C12Q 2537/143**

## Description

## FIELD OF INVENTION

The present invention relates to the spatial localization of barcode units, and spatial omics.

## BACKGROUND OF INVENTION

Omics approaches are powerful tools that enable in-depth characterization of a cell, or population of cells, by identifying and quantifying its biological components (e.g., RNA, proteins) in a dynamic manner.

There is growing interest in developing these approaches further when applied to tissues or other complex biological samples, in order to map the variations of the transcriptome, proteome, metabolome and the like, depending on the position of the cell in the sample. In other words, spatial omics aims to add spatial information to omics analysis.

Several approaches are readily available in the art for implementing spatial omics.

For example, Visium Spatial Gene Expression technology developed by 10x Genomics (see, *e.g.,* 10x Genomics, 2019, "Visium Spatial Gene Expression Solution"; or WO2021/102039) relies on oligonucleotides microarrays. Typically, a microarray is formed by "spotting" droplets comprising oligonucleotides on a slide; the position of each oligonucleotide is known, thus enabling recapitalization of the spatial information for the RNA captured by the oligonucleotide.

However, this approach is limited by the resolution of the microarray itself, due to local diffusion of the droplets during the spotting.

Other approaches aim to overcome this limitation. For exemple, Rodriques, et al. (Science vol. 363,6434 (2019): 1463-1467) describe a slide-seq approach wherein barcode beads are randomly deposited on a slide, and then indexed, *i.e.,* the barcodes are sequenced in order to associate an oligonucleotide to a given position on the slide. The slide is subsequently contacted with a sample (tissue slice); the captured analytes are then sequenced. Another example is Liao et al. 2023 (bioRxiv preprint doi: https://doi.org/10.1101/2023.04.28.538364) that describes a stereo-seq approach based on a similar method except for barcoded beads that are replaced with DNA nanoballs.

While these approaches improve resolution, they compulsorily involve, for each slide, indexing the position of each oligonucleotide on the slide with a first sequencing step, followed by a second sequencing once the analytes are captured, thereby complexifying the process.

The patent US, 11,624,088 provides an alternative, with beads comprising 2 types of barcoded oligonucleotides on the same bead: one type is responsible for capturing a analyte, and the other type is responsible for connecting beads together, thereby associating barcode information on 2 or more distinct beads and reconstituting relative spatial location of the barcoded bead array. This approach thus circumvents the need to perform 2 sequencing steps, as the spatial information is deduced from the connections between beads rather than by a preliminary indexing.

However, in this approach, nothing prevents the self-hybridization of the connection oligonucleotides. This is of importance because a significant part of the library produced for the sequencing of these barcode beads will not provide spatial information. Moreover, having 2 species of oligonucleotides on each bead increases manufacturing costs and complexity.

It is also worth mentioning another approach disclosed by Greenstreet et al. (bioRxiv preprint doi: https://doi.org/10.1101/2022.03.22.485380) called GPS-seq, based on a single layer of barcoded beads that is contacted with a slice of hydrogel containing spots (or satellites) of spatially indexed oligonucleotide, then contacted with a biological sample. The spatial indexes diffuse on to the beads from their initial spatial location in the hydrogel. Thus, neighborhood relationships are reconstituted based on shared spatial indexes.

This approach seems, however, technically challenging, and difficult to implement. In particular, the quality of the resolution is strongly dependent on the homogeneity of diffusion of the spatially indexed oligonucleotide, which is a parameter that is well known to be hard to control.

There is therefore an unmet need for an improved solution for spatial omics, in particular spatial transcriptomics, that is easy to manufacture and implement, while maintaining a high resolution. The present invention provides such a solution.

## SUMMARY

This invention thus relates to a composition comprising:

- a population of capture barcode units, wherein each capture barcode unit comprises a population of capture oligonucleotides, wherein each capture oligonucleotide comprises a barcode sequence, and a capture sequence capable of binding an analyte from a biological sample;
- a population of positioning barcode units, wherein each positioning barcode unit comprises a population of positioning oligonucleotides, wherein each positioning oligonucleotide comprises a barcode sequence, and a first connection sequence that can hybridize on a capture oligonucleotide;

wherein each barcode unit comprises a distinct barcode sequence, and all oligonucleotides on a same barcode unit comprise the same barcode sequence.

In some embodiments, the capture sequence on a first capture oligonucleotide cannot hybridize with the capture sequence of a second capture oligonucleotide, and wherein the first connection sequence on a first positioning oligonucleotide cannot hybridize with the first connection sequence of a second positioning oligonucleotide.

In some embodiments, the capture sequence cannot self-hybridize. In some embodiments, the first connection sequence cannot self-hybridize.

In some embodiments, the first connection sequence of the positioning oligonucleotides can hybridize on the capture sequence of the capture oligonucleotides.

In some embodiments, the capture oligonucleotides comprise a second connection sequence, wherein the second connection sequence is distinct from the barcode sequence and the capture sequence, and wherein the first connection sequence of the positioning oligonucleotides can hybridize on the second connection sequence of the capture oligonucleotides.

In some embodiments, the capture sequence is identical for all capture barcode units, and the first connection sequence is identical for all positioning barcode units.

In some embodiments, the capture sequence comprises a poly(dT) sequence or a poly(dU) sequence, and wherein the first connection sequence comprises a poly(dA) sequence.

In some embodiments, the capture oligonucleotides and/or the positioning oligonucleotides further comprise at least one PCR priming sequence.

In some embodiments, each oligonucleotide from the population of capture oligonucleotides and/or the population of positioning oligonucleotides further comprise at least one unique molecular identifier (UMI).

In some embodiments, the capture oligonucleotides and/or the positioning oligonucleotides further comprise at least one linker.

In some embodiments, the capture oligonucleotides comprise, in the 5'-P end to 3'-OH end order: optionally at least one linker, optionally at least one PCR priming sequence, the barcode sequence, optionally at least one UMI, and the capture sequence; and wherein the positioning oligonucleotides comprise, in the 5'-P end to 3'-OH end order: optionally at least one linker, optionally at least one PCR priming sequence, the barcode sequence, and the first connection sequence.

In some embodiments, the barcode units are synthetic barcode units.

In some embodiments, the barcode units are beads or DNA nanoballs.

In some embodiments, the capture barcode units and/or the positioning barcode units have distinct sizes.

In some embodiments, the population of capture barcode units and the population of positioning barcode units are assembled on a substrate.

In some embodiments, the capture barcode units are in contact with at least one, preferably at least 2, more preferably at least 3 positioning barcode units.

The present invention further relates to a method for determining the spatial location of a population of capture barcode units, comprising the steps of:

(i) contacting the population of capture barcode units with the population of positioning barcode units from the composition according to the invention;

(ii) forming a plurality of contact points between the populations of capture and positioning barcode units, wherein each contact point involves one capture oligonucleotide and one positioning oligonucleotide;

(iii) at the plurality of contact points formed at step (ii), hybridizing at least one connection sequence of the positioning oligonucleotide with at least one complementary sequence on the capture oligonucleotide, thereby obtaining a plurality of hybridized oligonucleotides;

(iv) extending the plurality of hybridized oligonucleotides obtained at step (iii), thereby obtaining a plurality of nucleic acid sequences comprising the barcode sequence of a capture barcode unit, and the barcode sequence of a positioning barcode unit;

(v) optionally, amplifying the plurality of nucleic acid sequences obtained at step (iv), thereby obtaining a plurality of amplicons;

(vi) sequencing the plurality of nucleic acid sequences obtained at step (iv) or the plurality of amplicons obtained at step (v);

(vii) deducing the neighborhood relationships between the capture barcode units and the positioning barcode units;

(viii) deducing the relative position of each capture barcode unit and/or positioning barcode unit; and

(ix) optionally, deducing the absolute position of each capture barcode unit and/or positioning barcode unit.

The present invention further relates to a method for determining the spatial location of a plurality of analytes in a biological sample comprising thereof, comprising the steps of:

(i) contacting the population of capture barcode units with the population of positioning barcode units from the composition according to the invention;

(ii) forming a plurality of contact points between the populations of capture and positioning barcode units, wherein each contact point involves one capture oligonucleotide and one positioning oligonucleotide;

(iii) at the plurality of contact points formed at step (ii), hybridizing at least one connection sequence with at least one complementary sequence on the capture oligonucleotide, thereby obtaining a plurality of hybridized oligonucleotides;

(iv) extending the plurality of hybridized oligonucleotides obtained at step (iii), thereby obtaining a plurality of nucleic acid sequences comprising the barcode sequence of a capture barcode unit, and the barcode sequence of a positioning barcode unit;

(v) optionally, amplifying the plurality of nucleic acid sequences obtained at step (iv), thereby obtaining a plurality of amplicons;

(vi) sequencing the plurality of nucleic acid sequences obtained at step (iv) or the plurality of amplicons obtained at step (v);

(vii) deducing the neighborhood relationships between the capture barcode units and the positioning barcode units;

(viii) deducing the relative position of each capture barcode unit and/or positioning barcode unit; and

(ix) optionally, deducing the absolute position of each capture barcode unit and/or positioning barcode unit.

and further comprising the steps of:

a) at any step before step (v), contacting the populations of capture and positioning barcode units with the biological sample;

b) at any step after step (a), and before step (v), capturing the plurality of analytes from the biological sample by binding the plurality of analytes to the capture sequence of a plurality of capture barcode units;

c) optionally, after step (b), extending the capture sequence on the sequence of the plurality of analytes, thereby obtaining a plurality of nucleic acids comprising the barcode sequence of a capture barcode unit and the sequence of the analyte;

d) optionally, after step (c), amplifying the plurality of nucleic acids, thereby obtaining a plurality of amplicons;

e) sequencing the plurality of nucleic acid sequences obtained at step (c) or the plurality of amplicons obtained at step (d);

f) at any step after step (viii), deducing the relative position of each analyte; and optionally, deducing the absolute position of each analyte in the biological sample.

In some embodiments, the biological sample is a tissue, a tissue section, a monolayer of cells, multiple layers of cells including organoids, or a single cell, including a syncytium.

**DEFINITIONS**

In this disclosure, any use of the singular forms **"a", "an"** or **"the"** includes the singular but also the plural references, unless the context clearly indicates otherwise. Thus, for example, a reference to "an agent" includes a single agent and a plurality of such agents.

In the present invention, the following terms have the following meanings:

**"About"** or **"approximately"** can mean within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. Alternatively, "about" preceding a figure means plus or less 10 % of the value of said figure. Alternatively, particularly with respect to biological systems or processes, the term can mean within an order of magnitude, within 5--fold, and more preferably within 2--fold, of a value. Where particular values are described in the application and claims, unless otherwise stated the term "about" meaning within an acceptable error range for the particular value should be assumed.

**"Amplification"** refers to the process of producing multiple copies, i.e., at least 2 copies, of a desired template sequence. Techniques to amplify nucleic acids are well known to the skilled artisan and include specific amplification methods as well as random amplification methods.

**"Barcode"** refers to a biomolecular sequence, such as a nucleic acid or amino acid sequence, preferably a nucleic acid sequence. A barcode can be attached to an analyte in a reversible or irreversible manner. It can be added to, *e.g.*, a fragment of a DNA or RNA molecule before and/or during sequencing, so as to convey information about, *e.g.*, the source or origin of the DNA or RNA molecule.

**"Barcode unit"** refers to a body comprising at least one copy of a barcode sequence. In one embodiment, the barcode unit comprises a core, a support or a substrate, and at least one oligonucleotide comprising the at least one copy of the barcode sequence, wherein the oligonucleotide is attached or bound to the core, support or substrate, typically the barcode unit is a barcoded bead. In another embodiment, the barcode unit consists of multiple repeats of a nucleic acid sequence comprising the barcode sequence, preferably consecutive repeats, typically the barcode unit is a DNA nanoball. In a preferred embodiment, the barcode unit has an overall spherical shape. In a preferred embodiment, the barcode unit is a synthetic barcode unit.

"Barcoding" refers to the attachment of a barcode, such as a nucleic acid barcode, to an analyte or a nucleic acid sequence, through primer template annealing, primer dependent DNA synthesis and/or ligation. In some embodiments, the analyte is a mRNA. In some embodiments, the nucleic acid sequence is an oligonucleotide, preferably a capture oligonucleotide or a positioning oligonucleotide according to the invention.

"Bead" refers to a particle that may be spherical (e.g., microspheres) or have an irregular shape. Beads may be as small as about 10 nm in diameter or as large as about several millimeters in diameter.

"library" refers to a library composed of complementary DNAs which are reverse transcribed from mRNAs.

"Clonal copies" refers to a population of identical copies of oligonucleotide of each barcode unit. Clonal copies may also be nearly identical, with sufficient identity, as the person skilled in the art will realize, to hybridize to identical sequences and/or achieve the same functions (e.g., priming, identification, etc.). In preferred embodiments, barcode sequence of two clonal copies share at least 70% sequence identity, preferably at least 80% sequence identity, preferably at least90 % sequence identity, preferably at least 93% sequence identity, and even more preferably at least 95% sequence identity. In preferred embodiments, at least 60%, preferably at least 70%, preferably at least 80%, preferably at least 90% and even more preferably at least 95% of clonal copies are identical.

"Complement" or "complementary" refer to a polynucleotide sequence capable of forming base pairing- by hydrogen bonds with another polynucleotide sequence, preferably Watson--Crick base -pairing unless explicitly specified otherwise. For example, guanine (G) is the Watson--Crick complementary base of cytosine (C), and adenine (A) is the Watson--Crick complementary base of thymine (T) and of uracil (U). Other types of base pairing including, without limitation, Wobble base pairing. For example, inosine (I) is a nucleoside which is capable of Wobble base pairing- with any natural base (C, T, U, A or G). Depending on the context, and as the person skilled in the art will clearly understand, "complementary" may mean "reverse complementary-",- *i.e.*, the sequences are complementary when read in opposite directions.

"Kit" or "kit-of-parts" refer to any manufacture (*e.g.*, a package or at least one container) comprising the different reagents necessary for carrying out the methods described herein, packed so as to allow their transport and storage. The terms "kit" and "kit-of-parts" shall encompass an entity of physically separated components, which are intended for individual use, but in functional relation to each other. A kit may be promoted, distributed, or sold as a unit for performing the methods described herein. Furthermore, any or all of the kit reagents may be provided within containers that protect them from the external environment, such as in sealed and sterile containers. The kit may also contain a package insert describing the kit and methods for its use.

"Lysis" refers to the disruption of a biological sample, in particular cells comprised in the biological sample, in order to gain access to materials that are otherwise inaccessible. Typically, lysis involves the rupture of the cell plasma membrane and may include the nuclear membrane. Lysis methods are well known to the skilled artisan, and include, but are not limited to, proteolytic lysis, chemical lysis, thermal lysis, mechanical lysis, and osmotic lysis.

"Nucleic acid sequence primer", or simply "primer", refers to an oligonucleotide that is capable of hybridizing or annealing with a nucleic acid and serving as an initiation site for nucleotide polymerization under appropriate conditions, such as the presence of nucleoside triphosphates and an enzyme for polymerization, such as DNA or RNA polymerase or reverse transcriptase, in an appropriate buffer and at a suitable temperature.

"Oligonucleotide" refers to a polymer of nucleotides, generally to a single -stranded polymer of nucleotides. In some embodiments, the oligonucleotide comprises from 2 to 500 nucleotides, preferably from 10 to 150 nucleotides, preferably from 20 to 100 nucleotides. Oligonucleotides may be synthetic or may be made enzymatically. In some embodiments, oligonucleotides may comprise ribonucleotide monomers, deoxyribonucleotide monomers, or a mix of both. As the skilled person will realize, nucleotide analogs (such as peptide nucleic acids, locked nucleic acids, glycol nucleic acids, etc.) may replace some or all of the nucleotides in an oligonucleotide.

"PCR priming sequence", "priming sequence" or "universal tag sequence" are interchangeable and refer to a nucleic acid sequence useful for enabling amplification, preferably PCR amplification and further sequencing of nucleic acid sequences extracted or derived from the biological units. In one embodiment, the PCR priming lacks homology with the template sequence.

"Polymerase chain reaction", abbreviated as "PCR", refers to methods including, but not limited to, allele specific- PCR, asymmetric PCR, hot-start PCR, intersequence specific- PCR, methylation -specific PCR, miniprimer PCR, multiplex ligation--dependent probe amplification, multiplex -PCR, nested PCR1 quantitative PCR, reverse transcription PCR and/or touchdown PCR. DNA polymerase enzymes suitable to amplify nucleic acids comprise, but are not limited to, Taq polymerase Stoffel fragment, Taq polymerase, Advantage DNA polymerase, AmpliTaq, AmpliTaq Gold, Titanium Taq polymerase, KlenTaq DNA polymerase, Platinum Taq polymerase, Accuprime Taq polymerase, Pfu polymerase, Pfu polymerase turbo, Vent polymerase, Vent exo- polymerase, Pwo polymerase, 9 Nm DNA polymerase, Therminator, Pfx DNA polymerase, Expand DNA polymerase, rTth DNA polymerase, DyNAzyme-EXT Polymerase, Klenow fragment, DNA polymerase I, T7 polymerase, SequenaseTM, Tfi polymerase, T4 DNA polymerase, Bst polymerase, Bca polymerase, BSU polymerase, phi-29 DNA polymerase and DNA polymerase Beta or modified versions thereof. In one embodiment, the DNA polymerase has a 3'to-5' proofreading, i.e., exonuclease, activity. In one embodiment, the DNA polymerase has a 5'to-3' proofreading, i.e., exonuclease, activity. In one embodiment, the DNA polymerase has strand displacement activity, i.e., the DNA polymerase causes the dissociation of a paired nucleic acid from its complementary strand in a direction 5'to3', in conjunction with, and close to, the template--dependent nucleic acid synthesis. DNA polymerases such as E. coli DNA polymerase I, Klenow fragment of DNA polymerase I, T7 or T5 bacteriophage DNA polymerase, and HIV virus reverse transcriptase are enzymes that possess both the polymerase activity and the strand displacement activity. Agents such as helicases can be used in conjunction with inducing agents that do not possess strand displacement activity in order to produce the strand displacement effect, that is to say the displacement of a nucleic acid coupled to the synthesis of a nucleic acid of the same sequence. Likewise, proteins such as Rec A or Single--Strand DNA Binding Protein (SSB) from E. coli or from another organism could be used to produce or to promote the strand displacement, in conjunction with other inducing agents.

**"Population"** or **"plurality"** refers to a group comprising at least 2 barcode units of the same species, type, or model. In one embodiment, all units in the population are identical. In another embodiment, units in the population may comprise minor structural differences (*e.g.*, a label, a tag, a unique unit identifier, or any suitable means to distinguish one unit from another within the population) but share the same function(s) and/or properties.

**"Primer--directed extension"** refers to any method known in the art wherein primers are used to initiate replication of nucleic acid sequences in the linear or logarithmic amplification of nucleic acid molecules. Primer--directed extension may be accomplished by any of several schemes known in this art including, but not limited to, polymerase chain reaction (PCR), ligase chain reaction (LCR) and strand -displacement amplification (SDA). "Primer-directed extension" can be carried out by DNA polymerase enzymes as described herein.

**"Random amplification techniques"** include, without limitation, multiple displacement amplification (MDA), random PCR, random amplification of polymorphic DNA (RAPD) or multiple annealing and looping based amplification cycles (MALBAC). By opposition, "specific amplification techniques" include without limitation, methods requiring temperature cycling (such as polymerase chain reaction (PCR), ligase chain reaction, transcription-based amplification) and/or isothermal amplification systems (such as self-sustaining sequence replication, replicase system, helicase system, strand displacement amplification, rolling circle--based amplification and NASBA).

**"Reverse transcription"** refers to the replication of RNA to produce DNA complementary strands DNA (cDNA), in particular using an RNA-directed DNA polymerase (such as, *e.g.*, reverse transcriptase, RT). The reverse transcription of RNAs may be carried out by techniques well known to the skilled artisan, using a reverse transcriptase enzyme and a mix of 4 deoxyribonucleotide triphosphates (dNTPs), namely deoxyadenosine triphosphate (dATP), deoxycytidine triphosphate (dCTP), deoxyguanosine triphosphate (dGTP) and (deoxy)thymidine triphosphate (dTTP). In some embodiments, the reverse transcription of RNAs comprises a first step of first strand cDNA synthesis. Methods for first strand cDNA synthesis are well known to the skilled artisan. First strand cDNA synthesis reactions can use a combination of sequence-specific primers, oligo(dT) primers or random primers. Examples of reverse transcriptatase enzymes include, but are not limited to, MMLV reverse transcriptase, SuperScript II (Invitrogen), SuperScript III (Invitrogen), SuperScript IV (Invitrogen), Maxima (ThermoFisher Scientific), ProtoScript II (New England Biolabs), PrimeScript (ClonTech). In some embodiments, the reverse transcription can be a primer-directed extension. In these embodiments, the reverse transcription requires a single-stranded DNA molecule comprising at least a probing sequence capable of hybridization with a portion of the RNA and serving as amplification primer site. The single-stranded DNA molecule may further contain other functional sequences. In some embodiments, the single-stranded DNA molecule is an oligonucleotide bound or otherwise attached to a particle as will be described herein, comprising *inter alia* a unique identifier such as a nucleic acid barcode. cDNA polymerization starts from the amplification primer site, and the resulting cDNA molecule (so called "first-strand cDNA molecule") contains (i) the original single stranded DNA molecule including the functional sequences when present and (ii) the complementary DNA sequence of the hybridized RNA.

**"Second-strand synthesis"** refers to the synthesis of a complementary sequence to the first strand cDNA molecule to produce an at least partially double- stranded DNA molecule. Second- -strand synthesis can be implemented to obtain cDNA replicates in solution without the need for an intermediate step such as an extraction step (e.g., in case of chemical contamination) or molecular cleavage (e.g., in case of a particle--grafted cDNA). In some embodiments, second -strand synthesis can be a primer -directed extension. In these embodiments, the second -strand synthesis requires a single stranded DNA molecule at least comprising- a probing sequence, at its 3'-end, capable of hybridization with a portion of the first -strand cDNA molecule and serving as amplification primer site. In some embodiments, these primers are random oligonucleotides. In some embodiments, the primers are specific to some target sequences. The single -stranded DNA molecule may further contain other functional sequences located in 5'-direction from the probing sequence, such as, e.g., unique molecular identifier (UMI), PCR priming and/or nucleic acid barcode. In these embodiments, second -strand synthesis starts from the amplification primer site and the resulting DNA molecule (the so -called "second -strand cDNA molecule") contains (i) the original single -stranded DNA molecule including the functional sequences when present and (ii) the complementary DNA sequence of the first -strand cDNA molecule. Methods for second strand cDNA synthesis are -well known- to the skilled artisan, and can be implemented using DNA--dependent DNA polymerases, such as, e.g., Taq polymerase Stoffel fragment, Taq polymerase, Advantage DNA polymerase, AmpliTaq, AmpliTaq Gold, Titanium Taq polymerase, KlenTaq DNA polymerase, Platinum Taq polymerase, Accuprime Taq polymerase, Pfu polymerase, Pfu polymerase turbo, Vent polymerase, Vent exo- polymerase, Pwo polymerase, 9 Nm DNA polymerase, Therminator, Pfx DNA polymerase, Expand DNA polymerase, rTth DNA polymerase, DyNAzyme-EXT Polymerase, Klenow fragment, DNA polymerase I, T7 polymerase, SequenaseTM, Tfi polymerase, T4 DNA polymerase, Bst polymerase, Bca polymerase, BSU polymerase, phi-29 DNA polymerase and DNA polymerase Beta or modified versions thereof. In some embodiments, the DNA--dependent DNA polymerase has a 3'to-5' proofreading activity. In some embodiments, the DNA--dependent DNA polymerase has a 5'to-3' proofreading activity. In some embodiments, the DNA--dependent DNA polymerase has a 5'to-3' exonuclease activity. In some embodiments, the DNA--dependent DNA polymerase has strand displacement activity, i.e., the DNA--dependent DNA polymerase causes the dissociation of a paired nucleic acid from its complementary strand in a direction from 5' towards 3', in conjunction with, and close to, the template--dependent nucleic acid synthesis. DNA--dependent DNA polymerase such as *E. coli* DNA polymerase I, Klenow fragment of DNA polymerase I, T7 or T5 bacteriophage DNA polymerase, and HIV reverse transcriptase are enzymes which possess both a polymerase activity and a strand displacement activity.

**"Spatial location"** or **"position"** refers to the position, or coordinates, of an object (*e.g.*, an analyte or a barcode unit) in a bidimensional or planar space (2D) or a tridimensional space (3D). A tridimensional space may be, non-imitatively, a volume or an overall planar surface comprising some degree of irregularities in Z axis (e.g., protrusions, holes, and the like). Alternatively, the position in a tridimensional space may refer to a 3D projection of 2D positions, typically a reconstituted or extrapolated volume from a pile of successive 2D information.

**"Template"** or **"template sequence"** refers to a nucleic acid sequence for which amplification is desired. A template can comprise DNA or RNA. The template sequence may be known or unknown.

## DETAILED DESCRIPTION

This invention relates to a composition comprising:

- a population of capture barcode units, wherein each capture barcode unit comprises a population of capture oligonucleotides, wherein each capture oligonucleotide comprises a barcode sequence, and a capture sequence capable of binding an analyte from a biological sample;
- a population of positioning barcode units, wherein each positioning barcode unit comprises a population of positioning oligonucleotides, wherein each positioning oligonucleotide comprises a barcode sequence, and a connection sequence that can hybridize on a capture oligonucleotide;

In a preferred embodiment, each barcode unit comprises a distinct barcode sequence, and all oligonucleotides on a same barcode unit comprise the same barcode sequence.

It will be understood that the present invention provides useful solutions for decoding the position of barcode units relative to one another. By combining this approach with the capture and detection of analytes (*e.g.*, mRNA) at high resolution, the present invention thereby enables decoding the position and distribution of analytes in a complex biological sample such as a tissue, or a section of such tissue. Therefore, the present application provides a useful approach for spatial multi-omics such as spatial transcriptomics, spatial genomics, and the like.

The present invention enables the capture of analytes from a biological sample while retaining the spatial information of their biological sample of origin and barcoding individually each analyte. This capture involves the use of a capture sequence, including but not limited to a nucleic acid sequence complementary to a sequence comprised on the analyte.

It will be apparent to the person skilled in the art that the present invention enables decoding the position of each barcode unit, and, by extension, each analyte, by recapitulating the neighborhood relationship between each barcode unit.

The solution of the invention relies on the use of 2 distinct populations of barcode units: a first population is dedicated to the capture and labelling of the analyte (capture barcode units), and a second population is dedicated to providing spatial information (positioning barcode unit). As such, each captured analyte is associated with both (i) information regarding the capture barcode unit it was associated with, and optionally a unique molecular identifier, and (ii) information regarding the neighborhood relationship between the capture barcode unit it was associated with and the surrounding positioning barcode units.

In some embodiments, the composition according to the invention enables spatial transcriptomics, i.e., detection of variations in gene expression depending on the position of a cell in a biological sample, typically a tissue.

In some embodiments, each capture barcode unit from the population of capture barcode unit comprises a population of capture oligonucleotides. As used herein, "population of capture oligonucleotides" means at least 100, 1.000, 10.000, 100.000, 1.000.000, 10.000.000, 100.000.000, or more capture oligonucleotides.

In some embodiments, each capture oligonucleotides comprise at least one capture sequence. In a preferred embodiment, each capture oligonucleotide comprises one capture sequence.

Within the scope of the present invention, the capture sequence acts as an analyte capture probe. In a preferred embodiment, the capture sequence is a nucleic acid sequence. In certain embodiments, the capture sequence is a DNA sequence or an RNA sequence. In a more preferred embodiment, the capture sequence is a DNA sequence.

In some embodiments, the capture sequence is single stranded or double stranded, preferably single stranded.

The capture sequence may be specifically designed for the capture of mRNA.

In a preferred embodiment, the capture sequence is configured for the nonspecific capture of nucleic acids, preferably mRNA. In certain embodiments, the capture sequence is capable of binding a poly(A) tail of mRNA. In some embodiments, the capture sequence comprises or consists of a poly(T) sequence.

In another, less preferred embodiment, the capture sequence is configured for the capture of specific target nucleic acids, preferably specific target mRNA molecules. In some embodiments, the capture sequence comprises or consists of a sequence that is complementary to a target sequence on the target nucleic acid. In some embodiments, the capture sequence is degenerate (i.e., with a random sequence) or specific of a nucleic acid sequence of interest.

In a preferred embodiment, the capture sequence can hybridize on the first connection sequence. In a preferred embodiment, the capture sequence of the capture oligonucleotides can hybridize on the first connection sequence of the positioning oligonucleotides.

In a preferred embodiment, the capture sequence cannot hybridize with the capture sequence. In some embodiments, the capture sequence on a first capture oligonucleotide on a capture barcode unit cannot hybridize with the capture sequence on a second capture oligonucleotide on the same capture barcode unit. In some embodiments, the capture sequence on a capture oligonucleotide on a first capture barcode unit cannot hybridize with the capture sequence on a capture oligonucleotide on a second capture barcode unit.

In some embodiments, the capture sequence cannot self-hybridize. In some embodiments, the capture sequence cannot form secondary structures with itself such as loops.

In a preferred embodiment, the capture sequence is identical for all barcode units from the population of capture barcode units.

In some embodiments, on capture oligonucleotides, the barcode sequence and the capture sequence are arranged in the 5'to 3' order.

In some embodiments, the capture sequence comprises or consists of a poly(dT) sequence or a poly(U) sequence. Thus, in some embodiments, the capture sequence is specific for a poly(dA) sequence. Poly(dA) sequences may be found, e.g., at the 3'end of mRNAs, within the poly-A tail.

In some embodiments, the capture sequence comprises or consists of the sequence (dT)nVN or (U) nVN (from 5' to 3'), wherein n ranges from 5 to 50, V represents any nucleotide but dT/U (i.e., dA, dC or dG), and N represents any nucleotide (i.e., dA, dT, U, dC or dG). Accordingly, in some embodiments, the capture sequence is specific of a NB(A)n sequence (from 5' to 3'), wherein n ranges from 5 to 50, B represents any nucleotide but dA (i.e., dT, U, dC or dG), and N represents any nucleotide (i.e., dA, dT, U, dC or dG). NB(A)n sequences may be found, e.g., at the 3'end of mRNAs, overlapping between the poly-A tail and the 3'UTR or CDS.

In some embodiments, the capture sequence comprises or consists of a polyinosine (poly(I)) sequence. Inosine is a nucleoside which is capable of Wobble base pairing with any natural base. In certain embodiments, the capture sequence is nonspecific and can prime to any nucleic acid sequence.

In some embodiments, the capture sequence of each capture oligonucleotide of a given capture barcode unit is one among a set of capture sequences, such set of capture sequences comprising at least 1 distinct capture sequences. In preferred such embodiments, the set of capture sequences comprises at least 2, at least 5, at least 10, at least 20, at least 50, or at least 100 distinct capture sequences. Such set of capture sequences allows, inter alia, for each given barcode unit to capture different sets of nucleic acid sequences from a given biological unit.

In some embodiments, the capture sequence of all capture oligonucleotides is identical. In some embodiments, the capture sequence is identical for all capture barcode units. In some embodiments, the capture sequence is identical for all capture oligonucleotides.

In some embodiments, the capture sequence comprises from 5 to 50 or more nucleotides. In some cases, the length of the capture sequence may be 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 nucleotides. In some cases, the length of the capture sequence may be at least 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 nucleotides or longer. In some cases, the length of the capture sequence may be at most 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 nucleotides or shorter.

In preferred embodiments, the length of the capture sequences may range from 5 to 100, preferably from 10 to 50, even more preferably from 20 to 40 nucleotides.

In some embodiments, each capture barcode unit can capture about 50%, 4°%, 30%, 30%, 10%, 5%, 2%, 1% of the total amount of the analyte in a cell.

In some embodiments, the capture oligonucleotides further comprise a second connection sequence.

In some embodiments, the first connection sequence can hybridize on the second connection sequence. In some embodiments, the first connection sequence of the positioning oligonucleotides can hybridize on the second connection sequence of the capture oligonucleotides.

In some embodiments, second connection sequence from a first capture oligonucleotide cannot hybridize with the second connection sequence of a second capture oligonucleotide. In some embodiments, the second connection sequence cannot self-hybridize. In some embodiments, the second connection sequence cannot hybridize with a capture sequence.

In some embodiments, the second connection sequence comprises from 5 to 50 or more nucleotides. In some embodiments, the second connection sequence is distinct from the barcode sequence and the capture sequence.

In some embodiments, each positioning barcode unit from the population of positioning barcode unit comprises a population of positioning oligonucleotides. As used herein, "population of positioning oligonucleotides" means at least 100, 1.000, 10.000, 100.000, 1.000.000, 10.000.000, 100.000.000, or more positioning oligonucleotides.

In some embodiments, each positioning oligonucleotides comprise at least one first connection sequence. In a preferred embodiment, each positioning oligonucleotide comprises one first connection sequence.

In some embodiments, the first connection sequence is single stranded or double stranded, preferably single stranded.

In a preferred embodiment, the first connection sequence can hybridize on the capture sequence. In a preferred embodiment, the first connection sequence of a preferred embodiment, the first connection sequence of the positioning oligonucleotides can hybridize on the capture sequence of the capture oligonucleotides.

In some embodiments, the first connection sequence cannot hybridize with the first connection sequence. In some embodiments, the first connection sequence on a first positioning oligonucleotide on a positioning barcode unit cannot hybridize with the first connection sequence on a second positioning oligonucleotide on the same positioning barcode unit. In some embodiments, the first connection sequence on a positioning oligonucleotide on a first positioning barcode unit cannot hybridize with the first connection sequence on a positioning oligonucleotide on a second positioning barcode unit.

In some embodiments, the first connection sequence cannot self-hybridize.

In some embodiments, on positioning oligonucleotides, the barcode sequence and the first connection sequence are arranged in the 5'to 3' order.

In some embodiments, the first connection sequence is identical for all barcode units from the population of positioning barcode units.

In some embodiments, the first connection sequence cannot bind at least one analyte from a biological sample.

In some embodiments, the capture sequence comprises a poly(dT) sequence or a poly(U) sequence, and the first connection sequence comprises a poly(dA) sequence.

In a preferred embodiment, each barcode unit comprises a distinct barcode sequence.

In a preferred embodiment, all oligonucleotides on a same barcode unit comprise the same barcode sequence. In certain embodiments, all capture oligonucleotides on a same capture barcode unit comprise the same barcode sequence. In certain embodiments, all positioning oligonucleotides on a same positioning barcode unit comprise the same barcode sequence.

In some embodiments, the number of clonal copies of a barcode sequence-is from about 2 to about 1012 clonal copies.

In some embodiments, the barcode sequence is preferably a non-optical identifier, in particular a nucleic acid barcode. In some embodiment, a nucleic acid barcode is single stranded or double stranded, preferably single stranded. In some embodiment, a nucleic acid barcode is a DNA barcode, an RNA barcode, or a mix of both.

In some embodiments, the first connection sequence is identical for all positioning barcode units. In some embodiments, the first connection sequence is identical for all positioning oligonucleotides.

In some embodiments, the capture sequence is identical for all capture barcode units, and the first connection sequence is identical for all positioning barcode units. In some embodiments, the capture sequence is identical for all capture oligonucleotides, and the first connection sequence is identical for all positioning oligonucleotides.

In some embodiments, a nucleic acid barcode can include from 5 to 20 or more nucleotides. In some cases, the length of a nucleic acid barcode may be 5, 6, 7, 8, 9, 10, 11, 12, 13, 14, 15, 16, 17, 18, 19, 20, 21, 22, 23, 24, 25, 26, 27, 28, 29, 30 nucleotides. In some cases, the length of a nucleic acid barcode may be at least 5, 6, 7, 8, 9, 10, 11, 12, 13, 14, 15, 16, 17, 18, 19, 20, 21, 22, 23, 24, 25, 26, 27, 28, 29, 30 nucleotides or longer. In some cases, the length of a nucleic acid barcode may be at most 5, 6, 7, 8, 9, 10, 11, 12, 13, 14, 15, 16, 17, 18, 19, 20, 21, 22, 23, 24, 25, 26, 27, 28, 29, 30 nucleotides or shorter. In preferred embodiments, the length of a nucleic acid barcode may be 5 to 20 nucleotides, preferably 8 to 16 nucleotides, 9 to 16 nucleotides and even more preferably 10 to 14 nucleotides. These nucleotides may be contiguous, i.e., in a single stretch of adjacent nucleotides, or they may be separated into two or more separate subsequences that are separated by 1 or more nucleotides.

In some embodiments, all the nucleic acid barcodes attached to a particular barcode unit include the same nucleic acid barcode sequence (*i.e.,* clonal copies of a nucleic acid barcode), but each barcode unit in a population of barcode units, or most barcode units in a population of barcode units, comprise a different nucleic acid barcode sequence, such that a large number of diverse nucleic acid barcode sequences are represented across a population of barcode units, e.g., at least about 1 000, 5 000, 10 000, 50 000, 100 000, 1 000 000, 5 000 000, 10 000 000, 50 000 000, 100 000 000, 500 000 000, 1 000 000 000 or more different nucleic acid barcode sequences.

In one embodiment, during utilization, the barcode units possess consistent sizes (uniform or nearly uniform).

In another embodiment, the barcode units possess varying sizes. In some embodiments, the barcode sequences of the capture oligonucleotides and the barcode sequences of the positioning oligonucleotides have distinct sizes.

In some embodiments, the positioning barcode units are not capable of binding an analyte.

In some embodiments, the positioning oligonucleotides does not comprise an analyte capture sequence.

In some embodiments, capture oligonucleotides and/or positioning oligonucleotides further comprise at least one PCR priming sequence. In some embodiments, the capture oligonucleotides and/or positioning oligonucleotides further comprise one PCR priming sequence. In other words, each capture oligonucleotide and each positioning oligonucleotide comprise at least one PCR priming sequence.

By **"PCR priming sequence",** it is meant a nucleic acid sequence useful for enabling amplification, preferably PCR amplification and further sequencing of nucleic acid sequences extracted or derived from a biological unit.

In some embodiments, the at least one PCR priming sequence comprises from 10 to 30 or more nucleotides. In some cases, the length of the PCR priming sequence may be 10, 11, 12, 13, 14, 15, 16, 17, 18, 19, 20, 21, 22, 23, 24, 25, 26, 27, 28, 29, 30 nucleotides. In some cases, the length of the PCR priming sequence may be at least 10, 11, 12, 13, 14, 15, 16, 17, 18, 19, 20, 21, 22, 23, 24, 25, 26, 27, 28, 29, 30, 31, 32, 33, 34, 34, 35, 36, 37, 38, 39, 40, 41, 42, 43, 44, 45, 46, 47, 48, 49, 50 nucleotides or longer. In some cases, the length of the at least one PCR priming sequence may be at most 10, 11, 12, 13, 14, 15, 16, 17, 18, 19, 20, 21, 22, 23, 24, 25, 26, 27, 28, 29, 30, 31, 32, 33, 34, 34, 35, 36, 37, 38, 39, 40, 41, 42, 43, 44, 45, 46, 47, 48, 49, 50 nucleotides or shorter.

In preferred embodiments, the length of at least one PCR priming sequence ranges from 15 to 35 nucleotides, preferably from 20 to 30 nucleotides, more preferably the length of the at least one PCR priming sequence is 25 nucleotides.

In a preferred embodiment, the at least one PCR priming sequence is identical in every oligonucleotide of a given capture barcode unit and/or positioning barcode unit; preferably the at least one PCR priming sequence is identical in every oligonucleotide of the population of capture barcode units and/or in every oligonucleotide of the population of positioning barcode units.

In one embodiment, at least one PCR priming sequence on the capture oligonucleotides is identical to at least one PCR priming sequence on the positioning oligonucleotides. In another embodiment, at least one PCR priming sequence on the capture oligonucleotides is distinct from at least one PCR priming sequence on the positioning oligonucleotides.

In some embodiments, the PCR priming sequence is a primer sequence.

In some embodiments, each oligonucleotide from the population of capture oligonucleotides and/or from the population of positioning oligonucleotides further comprise at least one unique molecular barcode (UMI). In other words, each capture oligonucleotide and/or each positioning oligonucleotide comprise at least one UMI.

By **"unique molecular identifier"** or **"UMI",** it is meant a nucleic acid sequence serving as an index, useful for reducing errors and quantitative bias introduced by amplification steps.

In a preferred embodiment, at least one UMI is different in each oligonucleotide of each barcode unit. In certain embodiments, at least one UMI is different in each capture oligonucleotide of each capture barcode unit. In certain embodiments, at least one UMI is different in each positioning oligonucleotide of each positioning barcode unit.

In some embodiments, the oligonucleotides of a capture barcode unit and/or the oligonucleotides of a positioning barcode unit comprise a wide variety of different UMIs, in particular more than 100, more than 1 000, more than 2 000, more than 5 000 or more than 10 000 different UMIs.

In some embodiments, the unique molecular identifier comprises from 3 to 30 or more nucleotides. In some cases, the length of the unique molecular identifier may be 3, 4, 5, 6, 7, 8, 9, 10, 11, 12, 13, 14, 15, 16, 17, 18, 19, 20, 21, 22, 23, 24, 25, 26, 27, 28, 29, 30 nucleotides. In some cases, the length of the unique molecular identifier may be at least 3, 4, 5, 6, 7, 8, 9, 10, 11, 12, 13, 14, 15, 16, 17, 18, 19, 20, 21, 22, 23, 24, 25, 26, 27, 28, 29, 30 nucleotides or longer. In some cases, the length of the unique molecular identifier may be at most 3, 4, 5, 6, 7, 8, 9, 10, 11, 12, 13, 14, 15, 16, 17, 18, 19, 20, 21, 22, 23, 24, 25, 26, 27, 28, 29, 30 nucleotides or shorter.

In preferred embodiments, the length of the unique molecular identifier ranges from 5 to 25 nucleotides, preferably from 7 to 15 nucleotides, more preferably from 8 to 13 nucleotides.

In one embodiment, each oligonucleotide from the population of capture oligonucleotides and/or from the population of positioning oligonucleotides comprise one UMI.

In another embodiment, each oligonucleotide from the population of capture oligonucleotides and/or from the population of positioning oligonucleotides comprise more than one UMI. The presence of several UMI on an oligonucleotide may be useful for the sequencing of the oligonucleotide.

In some embodiments, each oligonucleotide from the population of capture oligonucleotides and/or from the population of positioning oligonucleotides further comprises at least one linker. In other words, each capture oligonucleotide and/or each positioning oligonucleotide comprise at least one linker.

In some embodiments, the capture oligonucleotides comprise, in the 5' to 3' order: optionally at least one linker, optionally at least one PCR priming sequence, the barcode sequence, optionally at least one UMI, and the capture sequence. In some embodiments, capture oligonucleotides comprise, in the 5' to 3' order: at least one linker, at least one PCR priming sequence, the barcode sequence, at least one UMI, and the capture sequence. In some embodiments, capture oligonucleotides comprise, in the 5' to 3' order: at least one linker, at least one PCR priming sequence, the barcode sequence, at least one UMI, the second connection sequence, and the capture sequence.

In some embodiments, the positioning oligonucleotides comprise, in the 5' to 3' order: optionally at least one linker, optionally at least one PCR priming sequence, the barcode sequence, optionally at least one UMI, and the first connection sequence. In some embodiments, positioning oligonucleotides comprise, in the 5'to 3' order: at least one linker, at least one PCR priming sequence, at least one UMI, the barcode sequence, and the first connection sequence.

In some embodiments, the capture oligonucleotides comprise, in the 5' to 3' order: optionally at least one linker, optionally at least one PCR priming sequence, a barcode sequence, optionally at least one UMI, and the capture sequence; and the positioning oligonucleotides comprise, in the 5' to 3' order: optionally at least one linker, optionally at least one PCR priming sequence, a barcode sequence, optionally at least one UMI, and the first connection sequence.

In some embodiments, the capture oligonucleotides comprise, in the 5' to 3' order: a barcode sequence, and the capture sequence; and the positioning oligonucleotides comprise, in the 5' to 3' order: a barcode sequence, and the first connection sequence.

In some embodiments, the capture oligonucleotides comprise, in the 5' to 3' order: at least one PCR priming sequence, a barcode sequence, at least one UMI, and the capture sequence; and the positioning oligonucleotides comprise, in the 5' to 3' order: at least one PCR priming sequence, a barcode sequence, at least one UMI, and the first connection sequence.

In some embodiments, the density and spacing of the oligonucleotides coupled to the barcode unit itself, or core of the barcode unit, can be regulated by the linker molecule, ensuring an adequate number of sites for target capture. Linkers also serve the purpose of preventing unwanted structures (such as secondary or tertiary structures) from forming or causing folding of molecules on the body's surface. Consequently, the linker can be designed in different ways, either as a linear segment or as a branched segment (such as a dendrimer or other branched segment). Moreover, linker molecules can be configured for selective attachment, using specific functional groups tailored to particular chemistries. They can also be designed for activatable cleavage (similar to molecular scissors) to enable controlled release of captured target material from the core of the barcode unit. This controlled cleavage can be triggered by various mechanisms, including thermal cleavage, pH shifts, photocleaving, enzymatic cleavage, or other appropriate cleaving mechanisms.

As used herein, the term 'cleavage' pertains to the separation of the covalent backbone in a DNA molecule. Cleavage can be initiated through various means, such as enzymatic or chemical hydrolysis of a phosphodiester bond, among others. It is possible to achieve both single-stranded and double-stranded cleavage, with the latter occurring as a consequence of two separate single-stranded cleavage events. DNA cleavage can lead to the generation of either blunt ends or staggered ends. In specific embodiments, fusion polypeptides are employed to accomplish targeted double-stranded DNA cleavage.

In one embodiment, the capture oligonucleotide and/or the positioning oligonucleotide are bound to the core of the barcode unit, e.g., a bead. In another embodiment, the capture oligonucleotide and/or the positioning oligonucleotide are bound to each other in a linear sequence, e.g., a DNA nanoball.

The capture oligonucleotide and/or the positioning oligonucleotide may comprise from 5 to 300 or more nucleotides. In some cases, the length of the capture oligonucleotide and/or the positioning oligonucleotide may be 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300 nucleotides. In some cases, the length of the capture oligonucleotide and/or the positioning oligonucleotide may be at least 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300 nucleotides or longer. In some cases, the length of the capture oligonucleotide and/or the positioning oligonucleotide may be at most 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300 nucleotides or shorter.

It is to be understood that the composition according to the invention comprises two distinct populations of barcode units: capture barcode units, and positioning barcode units.

In a preferred embodiment, capture barcode units comprise only one type of oligonucleotides. In some embodiments, capture barcode units comprise only capture oligonucleotides. In some embodiments, capture barcode units do not comprise positioning oligonucleotides.

In a preferred embodiment, positioning barcode units comprise only one type of oligonucleotides. In some embodiments, positioning barcode units comprise only positioning oligonucleotides. In some embodiments, positioning barcode units do not comprise capture oligonucleotides.

These two types of barcode units may share a number of features as described hereinafter, but are functionally different. The capture barcode units and positioning barcode unit are capable of binding to each other. However, while the capture barcode units can bind analytes from a biological sample, the positioning barcode sequence cannot.

In some embodiments, each capture barcode unit comprises 100, 1.000, 10.000, 100.000, 1.000.000, 10.000.000, 100.000.000, or more, capture oligonucleotides. In some embodiments, each positioning barcode unit comprises 100, 1.000, 10.000, 100.000, 1.000.000, 10.000.000, 100.000.000, or more, positioning oligonucleotides.

In some embodiments, the barcode units may have any suitable shape or form.

In some embodiments, the barcode units are 3-dimensional barcode units.

In some embodiments, the barcode units have a spherical shape or a nonspherical (e.g., ellipsoidal, prismatic, polyhedral, amorphous, and the like). In some embodiments, the barcode units have a general spherical shape. In some embodiments, the barcode units may have the shape of a pelota or a ball. In some embodiments, the barcode units may have a regular or irregular form or shape.

In some embodiments, the barcode units are synthetic or nature barcode units.

In a preferred embodiment, the barcode units are synthetic barcode units. As used herein, "synthetic" means that the barcode is synthetized *in vitro.* In some embodiments, the barcode units are not produced by natural means (*e.g.*, a cell).

In another, less preferred embodiment, the barcode units are natural barcode units, *i.e.,* the barcode units are produced by natural means. In some embodiments, the barcode units are synthetized by a cell, *e.g.*, a bacterium.

In some embodiments, the barcode units are bodies comprising at least one copy of a barcode sequence.

In one embodiment, the barcode unit comprises a core, a support or a substrate, and a population of oligonucleotides, each comprising the at least one copy of the barcode sequence, wherein the oligonucleotides are attached or bound to the core, support or substrate.

In another embodiment, the barcode unit consists of multiple repeats of a nucleic acid sequence comprising the at least one barcode sequence, preferably consecutive repeats.

In some embodiments, the capture barcode units and/or positioning barcode units are beads or DNA nanoballs. In some embodiments, the capture barcode units and positioning barcode units are beads or DNA nanoballs. As used herein, "DNA nanoball" refers to a nucleic acid sequence comprising more than one repeat of the oligonucleotide as described herein.

In some embodiments, the capture barcode units are spots on a DNA microarray or slide. As used herein, "spots" refers to discrete locations on a DNA microarray or slide, typically designed to retain DNA molecules.

In some embodiments, the positioning barcode units are beads, DNA nanoballs, or spots on a DNA microarray.

In some embodiments, when positioning barcode units are beads, the oligonucleotides as defined herein are attached to the bead (*i.e.*, the core of the bead) by any suitable mean known in the art, *e.g.*, an anchor.

In some embodiments, when positioning barcode units are DNA nanoballs, the nucleic acid sequence of the DNA nanoball comprises the oligonucleotide, and therefore the barcode sequence itself.

In some embodiments, the beads (*i.e.,* the core of the beads) are made in a material selected from the group comprising acrylics, carbon, cellulose, ceramics, controlled-pore glass, cross-linked polysaccharides, gels, glass, gold, graphite, inorganic glasses, inorganic polymers, latex, metal oxides, metalloids, metals, mica, molybdenum sulfides, nanomaterials, nitrocellulose, optical fiber bundles, organic polymers, paper, plastics, polyacryloylmorpholide, poly(4-methylbutene), polyethylene terephthalate), poly(vinyl butyrate), polybutylene, polydimethylsiloxane, polyethylene, polyformaldehyde, polymethacrylate, polypropylene, polysaccharides, polystyrene, polyurethanes, polyvinylidene difluoride, quartz, rayon, resins, rubbers, semiconductor material, silica, silicon, sulfide, and combinations thereof.

In some embodiments, all or part of the barcode units are magnetic. In some embodiments, the barcode units are made of a magnetic material. In some embodiments, the capture barcode units are magnetic beads. In some embodiments, the positioning barcode units are magnetic beads.

In one embodiment, the capture barcode units and the positioning barcode units are made of different materials. In one embodiment, the capture barcode units and the positioning barcode units are made of the same material.

In some embodiments, the barcode units may be solid (or rigid, hard) or semi solid (or flexible, soft) barcode units.

In some embodiments, the capture barcode units and the positioning barcode units are both solid barcode units. In some embodiments, the capture barcode units and the positioning barcode units are both semi solid barcode units.

In a preferred embodiment, one type of barcode units is rigid while the other type of barcode units is semi-rigid. In one embodiment, the capture barcode units are rigid and the positioning barcode units are semi rigid. In another embodiment, the positioning barcode units are rigid and the capture barcode units are semi rigid.

In some embodiments, the barcode units from the population of capture barcode units and/or the barcode units from the population of positioning barcode units have distinct sizes (*i.e.*, polydisperse sizes).

In some embodiments, the barcode units from the population of capture barcode units and/or the barcode units from the population of positioning barcode units have identical sizes (*i.e.*, monodisperse sizes).

In some embodiments, the barcode units have a mean size ranging from about 1 nm to about 500 μm.

In some embodiments, the barcode units have a mean size ranging from about 10 nm to 100 μm.

In some embodiments, the barcode units have a mean diameter of about 1 μm, 2 μm, 3 μm, 4 μm, 5 μm, 6 μm, 7 μm, 8 μm, 9 μm, 10 μm, 11 μm, 12 μm, 13 μm, 14 μm, 15 μm, 16 μm, 17 μm, 18 μm, 19 μm, 20 μm, 21 μm, 22 μm, 23 μm, 24 μm, 25 μm, 26 μm, 27 μm, 28 μm, 29 μm, 30 μm, 31 μm, 32 μm, 33 μm, 34 μm, 35 μm, 36 μm, 37 μm, 38 μm, 39 μm, 40 μm, 41 μm, 42 μm, 43 μm, 44 μm, 45 μm, 46 μm, 47 μm, 48 μm, 49 μm, 50 μm.

In some embodiments, the barcode units have a mean size ranging from about 10 nm to 50 nm, preferably from 20 nm to 40 nm.

In some embodiments, the barcode units have a mean diameter of about 10 nm, 11 nm, 12 nm, 13 nm, 14 nm, 15 nm, 16 nm, 17 nm, 18 nm, 19 nm, 20 nm, 21 nm, 22 nm, 23 nm, 24 nm, 25 nm, 26 nm, 27 nm, 28 nm, 29 nm, 30 nm, 31 nm, 32 nm, 33 nm, 34 nm, 35 nm, 36 nm, 37 nm, 38 nm, 39 nm, 40 nm, 41 nm, 42 nm, 43 nm, 44 nm, 45 nm, 46 nm, 47 nm, 48 nm, 49 nm, 50 nm. In some embodiments, the barcode units have a mean diameter of about 20 nm, 21 nm, 22 nm, 23 nm, 24 nm, 25 nm, 26 nm, 27 nm, 28 nm, 29 nm, 30 nm, 31 nm, 32 nm, 33 nm, 34 nm, 35 nm, 36 nm, 37 nm, 38 nm, 39 nm, 40 nm.

It will be apparent to the person skilled in the art that the size of the barcode units may be adapted to the application and/or type of analyte.

In some embodiments, barcode units, preferably capture barcode units, with a size inferior to 1 μm are preferably used for intracellular and/or subcellular applications. In some embodiments, barcode units, preferably capture barcode units, with a size superior to 1 μm are preferably used for cell-scale and/or tissue-scale applications.

In some embodiments, barcode units, preferably capture barcode units, with a size inferior to 10 μm enable a high resolution of the spatial information of each analyte. In some embodiments, barcode units, preferably capture barcode units, with a size inferior to 1 μm enable an even higher resolution of the spatial information of each analyte.

In some embodiments, the population of positioning barcode units are bound, attached, or otherwise linked to a substrate, preferably a support.

In some embodiments, the substrate or support is a solid support. In some embodiments, the solid support may be made of any suitable material such as glass, polymers, plastic, metal, silica, or combinations thereof.

In some embodiments, the substrate or support is a planar or bidimensional support. In some embodiments, the planar or bidimensional support is selected from the group comprising or consisting of a slide, a coverslip, an array, a flask, a dish and the like, preferably a slide or a coverslip.

In some embodiments, the substrate or support is covered or coated with at least one material that enables binding or linkage between the substrate or support and the barcode unit. At least one material that enables binding or linkage may be of any suitable nature such as a polymer, a polypeptide, a sugar or the like, or any combinations thereof.

In some embodiments, the population of capture barcode units and the population of positioning barcode units are assembled. As used herein, "assembled" is intended to refer to the formation of a heterogenous structure comprising at least one capture barcode unit and at least one positioning barcode unit. Within the scope of the present invention, the term "assembled" can be interchangeably used with the terms "packed", "arranged", "distributed" and the like.

In some embodiments, the assembly is formed by connections between a plurality of capture barcode units and a plurality of positioning barcode units. In some embodiments, contact points are formed between capture barcode units and positioning barcode units. In one embodiment, a contact point involves the capture sequence of at least one capture oligonucleotide of a capture barcode unit, and the first connection sequence of at least one positioning oligonucleotide of a positioning barcode unit. In another embodiment, a contact point involves the second connection sequence of at least one capture oligonucleotide of a capture barcode unit, and the first connection sequence of at least one positioning oligonucleotide of a positioning barcode unit. In some embodiments, at least 1%, 2%, 3%, 4%, 5%, 10%, 20%, 30%, 40%, 50%, 60%, 70%, 80%, 90%, or 100% of capture oligonucleotides on a capture barcode unit are involved in forming a contact point with a positioning barcode unit. In some embodiments, about 1%, 2%, 3%, 4%, 5%, 10%, 20%, 30%, 40%, 50%, 60%, 70%, 80%, 90%, or 100% of capture oligonucleotides on a capture barcode unit are involved in forming a contact point with a positioning barcode unit. In some embodiments, at least 1%, 2%, 3%, 4%, 5%, 10%, 20%, 30%, 40%, 50%, 60%, 70%, 80%, 90%, or 100% of capture oligonucleotides on a positioning barcode unit are involved in forming a contact point with a capture barcode unit. In some embodiments, about 1%, 2%, 3%, 4%, 5%, 10%, 20%, 30%, 40%, 50%, 60%, 70%, 80%, 90%, or 100% of capture oligonucleotides on a positioning barcode unit are involved in forming a contact point with a capture barcode unit.

In some embodiments, the capture oligonucleotide and positioning oligonucleotide hybridize. In a preferred embodiment, the hybridization occurs at a contact point. It is to be understood that the hybridization binds or tethers together one capture barcode unit with one positioning barcode unit, thus ensuring physical connection between the barcode units comprised in the assembly. In some embodiments, at least 1%, 2%, 3%, 4%, 5%, 10%, 20%, 30%, 40%, 50%, 60%, 70%, 80%, 90%, or 100% of contact points are hybridized. In some embodiments, about 1%, 2%, 3%, 4%, 5%, 10%, 20%, 30%, 40%, 50%, 60%, 70%, 80%, 90%, or 100% of contact points are hybridized.

It will be understood that the modes of assembly listed hereinafter are not meant to be limitative but rather illustrative.

In some embodiments, the barcode units are assembled in one or more layers. As used herein, "one or more layers" comprises 1 layer (i.e., monolayer), 2 layers (i.e., bilayer), 3 layers, 4 layers, 5 layers, 10 layers, or more.

In one embodiment, each layer is homogeneous, i.e., one layer comprises more than 90%, 95%, 99%, 99.5%, or 100%, preferably 100%, of one type of barcode unit. In another embodiment, each layer is heterogeneous, i.e., one layer comprises both positioning barcode units and the capture barcode units. In certain embodiments, one or more layers comprises both homogeneous layers and heterogenous layers.

In some embodiments, one or more layers can adopt any suitable configuration. It will be apparent to the person skilled in the art which configuration can be used depending on the desired application.

In some embodiments, one or more layers can be configured with a one homogenous layer of positioning barcode units and another homogenous layer of capture barcode units. In some embodiments, one or more layers can be configured with 2, 3, 4 or more layers of heterogenous barcode units. In some embodiments, the one or more layers can be configured with a first homogenous layer of positioning barcode units, one or two layers of capture barcode units, and another layer of positioning barcode units

In one embodiment, the barcode units are assembled in random manner, ordered manner, or crystalline manner. The type of assembly may depend on the number of layers.

In some embodiments, the barcode units are assembled in random manner, i.e., the distribution of the positioning barcode units and the capture barcode units on the substrate is not controlled or random and may result in unequal distribution of the positioning barcode units and the capture barcode units on the substrate.

In some embodiments, the barcode units are assembled in ordered manner, i.e., the distribution of the positioning barcode units and the capture barcode units on the substrate is controlled and results in equal distribution of the positioning barcode units and the capture barcode units on the substrate. In some embodiments, when the barcode units are forming one or more layers, layers can be heterogenous (i.e., one layer comprises both positioning barcode units and the capture barcode units), or homogeneous (i.e., one layer comprises more than 90%, 95%, 99%, 99.5%, or 100%, preferably 100%, of one type of barcode unit). As such, in some embodiments, the barcode units are assembled in order to maximize the chances of contact between capture barcode units and positioning barcode units.

In some embodiments, the barcode units are assembled in a crystalline manner, i.e., the positioning barcode units and the capture barcode units are held together in an ordered, three-dimensional arrangement.

It is to be understood that the purpose of assembling the two types of barcode units (positioning and capture) together is to enable their positioning relative to one another. This step thus requires that the barcode units of different types are connected together. As such, it is to be understood that a high number of connections facilitates the reconstitution of neighborhood relationships between the barcode units.

In some embodiments, at least 10%, 20%, 30%, 40%, 50%, 60%, 70%, 80%, 90%, or 100% of barcode units from the population of capture barcode units are in contact with at least one, preferably at least 2, more preferably at least 3, positioning barcode units. In some embodiments, barcode units from the population of capture barcode units are in contact with at least one, preferably at least 2, more preferably at least 3, positioning barcode units. In a preferred embodiment, at least 10%, 20%, 30%, 40%, 50%, 60%, 70%, 80%, 90%, or 100% of barcode units from the population of capture barcode units have at least one hybridized oligonucleotide with at least one, preferably at least 2, more preferably at least 3, positioning barcode units. In a preferred embodiment, at least 10%, 20%, 30%, 40%, 50%, 60%, 70%, 80%, 90%, or 100% of the at least one hybridized oligonucleotide are sequenced.

Hence, in some embodiments, the contacts and/or physical proximity between capture barcode units and positioning barcode units is facilitated by at least one external force. Non limitative examples of such forces include mechanical forces, pressure, gravity, centrifuge force, clamping, magnetism, and the like. In some embodiments, at least one external force increases the probability, or ensures, that a capture barcode unit and a positioning barcode unit that are in close spatial proximity are in contact.

In some embodiments, the external force is gravity, i.e., the barcode units are sedimented. In some embodiments, the external force is centrifuge force, i.e., the barcode units are centrifuged at a suitable speed. In some embodiments, the external force is clamping. In some embodiments, the external force is magnetic force, i.e., the barcode units are magnetic themselves or pulled together by a magnetic field.

In some embodiments, the population of capture barcode units and the population of positioning barcode units are assembled on a substrate.

In one embodiment, the substrate is a support, i.e., it provides a solid mechanical support to the barcode units. In some embodiments, the support may be planar (i.e., bidimensional) or tridimensional.

In some embodiments, the substrate is a support, preferably a planar support. In some embodiments, the support has a smooth or rough surface. In some embodiments, the support has a homogeneous or heterogeneous surface. In some embodiments, the support comprises irregularities or structures on its surface, such as microwells, microarrays, scaffolds, or the like. It is to be understood that such structures aim to, e.g., position, retain, or expose the barcode units of the composition according to the invention. In some embodiments, the support comprises on its surface at least one means to bind the positioning barcode units and/or the positioning barcode units according to the invention.

In some embodiments, the support is a slide, an array, a chip, and the like. In some embodiments, the support is made in a material selected from the group comprising or consisting of glass, carbon, ceramics, metal, nitrocellulose, polymers, plastic, silicate, and combinations thereof.

In one embodiment, the support is rigid.

In another embodiment, the support is flexible, *i.e.*, the support can be folded. This embodiment is useful for application on a thick biological sample, wherein the support can be folded over or around the biological sample.

In some embodiments, the support has a size from about 1 mm to about 10 cm. In some embodiments, the support has a size of about 1 mm, 2 mm, 3 mm, 4 mm, 5 mm, 6 mm, 7 mm, 8 mm, 9 mm, 10 mm, 2 cm, 3 cm, 4 cm, 5 cm, 6 cm, 7 cm, 8 cm, 9 cm, 10 cm, or more. In some embodiments, the support has a surface of about 1 mm², 2 mm², 3 mm², 4 mm², 5 mm², 6 mm², 7 mm², 8 mm², 9 mm², 1 cm², 2 cm², 3 cm², 4 cm², 5 cm², or more.

In some embodiments, the substrate is a tridimensional substrate or support. In one embodiment, the substrate is a matrix. Non-limitative examples of matrices include hydrogels, collagen, and the like.

In some embodiments, the assembly of barcode units occupies about 1%, 2%, 3%, 4%, 5%, 6%, 7%, 8%, 9%, 10%, 20%, 30%, 40%, 50%, 60% ,70%, 80% or more of the total surface or volume of the substrate or support. In some embodiments, the assembly of barcode units occupies about 1 mm², 2 mm², 3 mm², 4 mm², 5 mm², 6 mm², 7 mm², 8 mm², 9 mm², 1 cm², 2 cm², 3 cm², 4 cm², 5 cm², or more of the total surface of the substrate or support.

In some embodiments, the composition according to the invention, when assembled as described herein, comprises a definite ratio of positioning barcode units and capture barcode units. It is to be understood that the ratio reflects the relative number of one type of barcode unit compared to the other type of barcode unit. This ratio may also be referred to as the level or degree of heterogeneity.

In one embodiment, the ratio of positioning barcode units to capture barcode units is about 1:2, 1:3, 1:4, 1:5, 1:6, 1:7, 1:8, 1:9, 1;10, 1:20, 1:30, 1:40, 1:50, 1:100, or more. In another embodiment, the ratio of capture barcode units to positioning barcode units is about 1:2, 1:3, 1:4, 1:5, 1:6, 1:7, 1:8, 1:9, 1;10, 1:20, 1:30, 1:40, 1:50, 1:100, or more.

In certain embodiments, the ratio of positioning barcode units to capture barcode units is about 1: 1.

In one embodiment, the composition or assembly comprises about 1.5-fold, 2-fold, 3-fold, 4-fold, 5-fold, 6-fold, 7-fold, 8-fold, 9-fold, 10-fold, 15-fold, 20-fold, 30-fold, 40-fold, 50-fold, 100-fold, 200-fold, 500-fold, 1 000-fold, 10 000-fold more positioning barcode units than capture barcode units. In another embodiment, the composition or assembly comprises about 1.5-fold, 2-fold, 3-fold, 4-fold, 5-fold, 6-fold, 7-fold, 8-fold, 9-fold, 10-fold, 15-fold, 20-fold, 30-fold, 40-fold, 50-fold, 100-fold, 200-fold, 500-fold, 1 000-fold, 10 000-fold more capture barcode units than positioning barcode units.

In some embodiments, the degree of heterogeneity is adjusted depending on the application and/or type of assembly. Such adjustments can be readily envisioned by the person skilled in the art. It will also be apparent to the person skilled in the art that the degree of heterogeneity may be adapted depending on the relative size of the barcode units.

As such, in some embodiments, when the barcode units are assembled in a random manner, the ratio of positioning barcode unit to capture barcode units is preferably about 1:1, *i.e.,* the number of barcode units of each type is equal or near equal. In some embodiments, when the barcode units are assembled in an ordered or crystalline manner, the ratio of positioning barcode unit to capture barcode units is different from 1:1, *i.e.,* one type of barcode unit is more represented than the other.

In some embodiments, the assembly of the barcode units is performed by any suitable technique selected from the group comprising or consisting of pick-and-place processes, self-assembly, sedimentation, creaming, entropy-driven colloidal assembly, solvent evaporation-assisted deposition, spin-coating, dipcoating, electrostatic deposition, electrophoretic deposition, capillary assembly, topographical-templated assembly, wettability-templated assembly, chemically-templated assembly, magnetic assembly, or any combination thereof. It will be apparent for the person skilled in thar art which technics are suitable depending on the expected results.

It is to be understood that the total number of barcode units may be adjusted depending on the application, the size of the barcode units, the density of the analytes and/or the biological sample. In some embodiments, the number of both capture barcode units and positioning barcode units is adjusted. In some embodiments, the number of capture barcode units is adjusted. In some embodiments, the number of positioning barcode units is adjusted.

In some embodiments, the total number of positioning barcode units is kept to the minimal number required, in particular required for the capture of positioning barcode units. In some embodiments, the total number of capture barcode units is kept at a higher level, *i.e.,* in order to capture the highest amount of analytes as possible.

In some embodiments, the number of capture barcode units is inversely proportional to the size of the capture barcode units (*i.e.,* when the size of the capture barcode units is decreased, the number of capture barcode units is increased; and when the size of the capture barcode units is increased, the number of capture barcode units is decreased).

Thus, in some embodiments, when the capture barcode units have a sub micrometer size, the number of capture barcode units is increased 2-fold, 3-fold, 4-fold, 5-fold, 6-fold, 7-fold, 8-fold, 9-fold, 10-fold, or more, compared to when the capture barcode units have a size above 1 $\mu$m.

It will be apparent to the person skilled in the art that the number of capture oligonucleotides in barcode units in the population of barcode units may be adapted to the number and/or density of analytes present in the biological sample, by adjusting the number of capture oligonucleotides comprised in each capture barcode units for efficient capture of the analytes. In some embodiments, the number of capture oligonucleotides comprised in each capture barcode units is increased when the density of analytes is high; in some embodiments, the number of capture oligonucleotides comprised in each capture barcode units is decreased when the density of analytes is low. As used herein, "high" means a 2-fold, 4-fold, 10-fold increase or more of the concentration of analytes compared to the average concentration in a biological sample. As used herein, "low" means a 2-fold, 4-fold, 10-fold decrease or more of the concentration of analytes compared to the average concentration in a biological sample.

In some embodiments, when the sample comprises a low density of analytes, the number of capture barcode units is increased 2-fold, 3-fold, 4-fold, 5-fold, 6-fold, 7-fold, 8-fold, 9-fold, 10-fold, or more.

In some embodiments, the population of barcode units comprises at least about 1 000, 5 000, 10 000, 50 000, 100 000, 1 000 000, 5 000 000, 10 000 000 or more barcode units.

In one embodiment, the ratio of analyte per capture barcode oligonucleotide is about 1:1, about 2:1, about 5:1, about 10:1, about 100:1, or more, preferably about 10:1. In some embodiments, the barcode units are at least 2-fold, 5-fold, 10-fold, 50-fold, 100-fold, or 1000-fold less numerous than the analytes.

In another embodiment, the ratio of capture barcode oligonucleotide per analyte is about 1:1, about 2:1, about 5:1, about 10:1, about 100:1, or more, preferably about 10:1. In some embodiments, the barcode units are at least 2-fold, 5-fold, 10-fold, 50-fold, 100-fold, or 1000-fold more numerous than the analytes.

It will be apparent to the person skilled in the art that increasing the concentration of capture barcode oligonucleotides and/or increasing the number of capture oligonucleotides per capture barcode unit increases the probability that a capture oligonucleotide binds to an analyte.

In some embodiments, the population of barcode units comprises at least 1000 barcode units. In some embodiments, the population of barcode units comprises at least 10 000 barcode units. In some embodiments, the population of barcode units comprises at least 100 000 barcode units. In some embodiments, the population of barcode units comprises at least 1 000 000 barcode units.

In some embodiments, the assembly of capture barcode units and positioning barcode units as described herein is stable. As used herein, "stable" means that the assembly is not altered, or substantially altered, by external and/or internal constraints, insults and/or stresses.

In some embodiments, the assembly of capture barcode units and positioning barcode units as described herein is stable over time, *i.e.,* it is not altered, or not substantially altered, after 1 hour, 1 day, 1 week, 1 month, 1 year, or more.

In some embodiments, the assembly of capture barcode units and positioning barcode units as described herein is stable when exposed to external insults such as mechanical, temperature, and the like. In some embodiments, the assembly of capture barcode units and positioning barcode units as described herein is stable when exposed to a mechanical insult, such as a mechanical shock, vibration, friction, and the like. In some embodiments, the assembly of capture barcode units and positioning barcode units as described herein is thermally stable, i.e., it remains stable at a temperature up to 10°C, 20°C, 30°C, 35°C, 37°C, 40°C, 50°C, 60°C, 70°C, 80°C, 90°C, or more.

In a preferred embodiment, the assembly of capture barcode units and positioning barcode units as described herein is stable upon storage. In some embodiments, the assembly of capture barcode units and positioning barcode units as described herein is stored in frozen state. By "frozen-state", it means maintained at a temperature from -200°C to 0°C, from -200°C to -20°C, from -200°C to -80°C, or at about -200°C, about -196°C, about -150°C, about -80°C, or about -20°C.

In a preferred embodiment, the assembly of capture barcode units and positioning barcode units as described herein is stable upon shipment and/or transportation by conventional means, i.e., in a suitable package, that may or may not be refrigerated by conventional means (e.g., dry ice) and transported by land, air or sea.

In some embodiments, the assembly of capture barcode units and positioning barcode units as described herein further comprises one or more components to increase stability.

In some embodiments, the one or more component may be any suitable molecule or mix of molecules that increases robustness of the assembly. In some embodiments, the one or more component is selected from the group comprising a hydrogel of any kind, preferably a hydrogel that can be depolymerized, a thickening agent or any agent that increases viscosity such as polyethylene glycol (PEG), pectin, gelatin, agar, cellulosic, gums, sugars, peptides or other polymers, or a frozen liquid, optionally comprising a cryoprotective agent.

In one embodiment, the population of capture barcode units and the population of positioning barcode units are stored separately, i.e., in separate containers, tubes, vials or the like. It is to be understood that, for applications that require a heterogenous ratio of capture barcode units to positioning barcode units, it is more convenient to have separate containers in order to adjust the ratio as desired.

In another embodiment, the population of capture barcode units and the population of positioning barcode units are stored in the same container, tube or vial, wherein the two populations of barcode units are maintained in conditions that prevent hybridization. In some embodiments, conditions that prevent hybridization may be by any suitable means such as a chemical, freezing, retention on another support, or the like. In a preferred embodiment, conditions that prevent hybridization are reversible, in particular, conditions that prevent hybridization can be reversed prior to the use of the composition according to the invention. It is to be understood that for applications that require a 1:1 ratio of capture barcode units to positioning barcode units, it is more convenient to have a composition with a 1:1 ratio readily available in one container.

In some embodiments, the area of contact between a capture barcode unit and a positioning barcode unit has a surface equivalent to 1/4, 1/8, 1/16, 1/20, 1/30, 1/40, 1/50, 1/60, 1/70, 1/80, 1/90, 1/100, 1/110, 1/120, 1/130, 1/140, 1/150, 1/160, 1/170, 1/180, 1/190, 1/200, or less, of the total surface of the barcode units. In some embodiments, the area of contact between a capture barcode unit and a positioning barcode unit has a surface equivalent to at least 1/200, at least 1/100, at least 1/50 of the total surface of the barcode units.

In some embodiments, the area of contact between a capture barcode unit and a positioning barcode unit has a surface of at least 1 nm$^2$, 5 nm$^2$, 10 nm$^2$, 50 nm$^2$, 100 nm$^2$, 500 nm$^2$, 1 $\mu$m$^2$, 5 $\mu$m$^2$, 10 $\mu$m$^2$, 50 $\mu$m$^2$, 100 $\mu$m$^2$, or more. In some embodiments, the area of contact between a capture barcode unit and a positioning barcode unit has a surface of at most 500 $\mu$m$^2$, 100 $\mu$m$^2$, or 50 $\mu$m$^2$.

In some embodiments, the area of contact between a capture barcode unit and a positioning barcode unit has a surface from 1 nm$^2$ to 100 $\mu$m$^2$, preferably from 10 nm$^2$ to 10 $\mu$m$^2$.

The present invention further relates to a substrate comprising the composition according to the invention, wherein the population of capture barcode units and the population of positioning barcode units are assembled on the substrate as described herein.

In some embodiments, the biological sample is selected from the group comprising or consisting of a tissue, a tissue section, a monolayer of cells, multiple layers of cells, an organoid, a syncytium or a single cell.

In some embodiments, the analyte is released from the biological sample. It is to be understood that the analyte must be readily accessible to the composition according to the invention, in particular, accessible to the capture barcode units.

In some embodiments, releasing the biological unit's analytes comprises a step of lysing the biological sample, or cells composing thereof.

In some embodiments, lysing the biological sample, or otherwise releasing the analytes from the biological sample, includes disrupting, permeabilizing or solubilizing the lipid membrane (e.g., the lipid bilayer) of the biological sample.

Means and methods for lysing the biological sample or otherwise releasing analytes from biological sample are well known in the art. In some embodiments, the method is chemical or mechanical. In some embodiments, the method is selected from the group comprising or consisting of chemical lysis (*e.g.*, using one or a combination of a detergent, a chaotropic agent, an alkaline agent, a solvent, etc.), physical lysis (*e.g.*, by one or a combination of heating, one or more freezing/thawing cycle, by osmotic shock, by cavitation, by sonication, etc.), and enzymatic lysis (*e.g.*, using enzymes).

In some embodiments, the analyte is selected from the group comprising or consisting of nucleic acids, amino acids, and small molecules. In some embodiments, the analyte is selected from the group comprising or consisting of DNA, RNA, miRNA, peptides or proteins, and small molecules.

Non limitative examples of DNA include genomic DNA, organellar DNA, i.e., mitochondrial DNA or chloroplast DNA. Non-limitative examples of RNA include messenger RNA, transfer RNA, ribosomal RNA, , microRNA, small interfering RNA, Piwi interacting RNA, antisense RNA, small nuclear RNA, small nucleolar RNA, small Cajal body RNA and enhancer RNA.

In some embodiments, the analyte is a nucleic acid molecule, preferably a nucleic acid molecule selected from the group comprising or consisting of DNA, RNA, and miRNA.

In some embodiments, the analyte is an RNA. In some embodiments, the analyte is a messenger RNA (mRNA), transfer RNA (tRNA), or ribosomal RNA (rRNA). In a preferred embodiment, the analyte is an mRNA.

In some embodiments, the analyte is DNA.

In one embodiment, the analyte is a single analyte. In another embodiment, the analyte is a multianalyte, i.e., the analyte comprises more than one type of molecule (e.g., a DNA molecule and a peptide), optionally the molecules are bound together.

In some embodiments, the analyte is a nucleic acid bound to another molecule selected from the group comprising or consisting of peptides, polypeptides, proteins, antibodies, small molecules, sugars, lipids, and combinations thereof. In some embodiments, the nucleic acid is a tag or label for another molecule such as peptides, polypeptides, proteins, antibodies, small molecules, sugars, and lipids. By "label" or "tag", it means attaching or linking, either covalently or non-covalently, the nucleic acid to the analyte. In some embodiments, labelling may comprise, *e.g.*, annealing, hybridizing, ligating, etc. the nucleic acid to the analyte.

In some embodiments, the step of releasing the analytes from the biological sample, and optionally lysing the biological sample, is carried out in a substrate or on a support, as described herein.

In some embodiments, the step of releasing the analytes from the biological sample, and optionally lysing the biological sample, includes or is followed by a sub-step of freezing the biological sample and/or the analytes released from the biological sample. By freezing, it is meant putting at a temperature from -200°C to 0°C, from -200°C to -20°C, from -200°C to -80°C, or at about -200°C, about - 196°C, about -80°C, or about -20°C. In some embodiments, the biological sample and/or the analytes released from the biological sample are flash-frozen (*e.g.*, with liquid nitrogen, dry ice, and the like).

In some embodiments, mRNA analyte bound to the capture sequence is reverse-transcribed, thereby forming a complementary DNA (cDNA).

The present further relates to a method for determining the spatial location of a population of capture barcode units, comprising the steps of:

(i) contacting the population of capture barcode units with the population of positioning barcode units from the composition according to the invention;

(ii) forming a plurality of contact points between the populations of capture and positioning barcode units, wherein each contact point involves one capture oligonucleotide and one positioning oligonucleotide;

(iii) at the plurality of contact points formed at step (ii), hybridizing at least one connection sequence with at least one complementary sequence on said capture oligonucleotide, thereby obtaining a plurality of hybridized oligonucleotides;

(iv) extending the plurality of hybridized oligonucleotides obtained at step (iii), thereby obtaining a plurality of nucleic acid sequences comprising both the barcode sequence of a capture barcode unit and the barcode sequence of a positioning barcode unit;

(v) optionally, amplifying the plurality of nucleic acid sequences obtained at step (iv), thereby obtaining a plurality of amplicons;

(vi) sequencing the plurality of nucleic acid sequences obtained at step (iv) or the plurality of amplicons obtained at step (v);

(vii) deducing the neighborhood relationships between the capture barcode units and the positioning barcode units;

(viii) deducing the relative position of each capture barcode unit and/or positioning barcode unit; and

(ix) optionally, deducing the absolute position of each capture barcode unit and/or positioning barcode unit.

In one embodiment, the complementary sequence is the capture sequence or a fraction thereof.

In another embodiment, the complementary sequence is a second connection sequence or a fraction thereof. In some embodiments, the second connection sequence is localized on the capture oligonucleotide. In some embodiments, the positioning oligonucleotide does not comprise the second connection sequence.

In some embodiments, step (i) and step (ii) are performed concomitantly.

In some embodiments, step (i) is performed prior to step (ii).

In some embodiments, at step (i), the population of capture barcode units and the population of positioning barcode units are added concomitantly.

In one embodiment, at step (i), the population of capture barcode units is added prior to the population of positioning barcode units. In another embodiment, at step (i), the population of positioning barcode units is added prior to the population of capture barcode units.

In some embodiments, at step (i), at least one layer is formed by the population of capture barcode units, and at least one layer is formed by the population of positioning barcode units.

In some embodiments, the biological sample is a tissue, a tissue section, a monolayer of cells, or multiple layers of cells.

In some embodiments, the plurality of contact points is formed between the populations of capture and positioning barcode units, wherein each contact point involves one capture oligonucleotide and one positioning oligonucleotide.

In one embodiment, a contact point involves the capture sequence of at least one capture oligonucleotide of a capture barcode unit, and the first connection sequence of at least one positioning oligonucleotide of a positioning barcode unit.

In another embodiment, a contact point involves the second connection sequence of at least one capture oligonucleotide of a capture barcode unit, and the first connection sequence of at least one positioning oligonucleotide of a positioning barcode unit.

In some embodiments, at least 1%, 2%, 3%, 4%, 5%, 10%, 20%, 30%, 40%, 50%, 60%, 70%, 80%, 90%, or 100% of capture oligonucleotides on a capture barcode unit are involved in forming a contact point with a positioning barcode unit. In some embodiments, about 1%, 2%, 3%, 4%, 5%, 10%, 20%, 30%, 40%, 50%, 60%, 70%, 80%, 90%, or 100% of capture oligonucleotides on a capture barcode unit are involved in forming a contact point with a positioning barcode unit.

In some embodiments, at least 1%, 2%, 3%, 4%, 5%, 10%, 20%, 30%, 40%, 50%, 60%, 70%, 80%, 90%, or 100% of capture oligonucleotides on a positioning barcode unit are involved in forming a contact point with a capture barcode unit. In some embodiments, about 1%, 2%, 3%, 4%, 5%, 10%, 20%, 30%, 40%, 50%, 60%, 70%, 80%, 90%, or 100% of capture oligonucleotides on a positioning barcode unit are involved in forming a contact point with a capture barcode unit.

In some embodiments, each capture barcode unit forms at least one, preferably at least 2, more preferably at least 3 contact points with distinct positioning barcode units. In some embodiments, each capture barcode unit is connected to at least 2, more preferably at least 3 distinct positioning barcode units. In some embodiments, each capture barcode unit forms at least one contact point with at least one, preferably at least 2, more preferably at least 3 positioning barcode units. In some embodiments, each capture barcode unit forms one contact point with at least one, preferably at least 2, more preferably at least 3 positioning barcode units. In some embodiments, each capture barcode unit forms at least one contact point with 1, 2, 3, 4, 5, 6, 7, 8, 9 or more positioning barcode units. In some embodiments, each capture barcode unit forms one contact point with 1, 2, 3, 4, 5, 6, 7, 8, 9 or more positioning barcode units. As used herein, "at least one" means 1, 2, 3, 4, 5, 6, 7, 8, 9, 10, or more.

It is to be understood that the above-mentioned number of contact points represents an average number over the whole population of barcode units.

In some embodiments, 10%, 20%, 30%, 40%, 50%, 60%, 70%, 80%, 90%, 95%, 99%, 99.5%, or 100% of the capture barcode units have formed at least one contact point with at least one positioning barcode unit.

In some embodiments, 10%, 20%, 30%, 40%, 50%, 60%, 70%, 80%, 90%, 95%, 99%, 99.5%, or 100% of the positioning barcode units have formed at least one contact point with at least one capture barcode unit.

It is to be understood that the populations of capture barcode units and positioning barcode units are considered as being connected or assembled as long as a substantial proportion of the barcode units are connected as described hereinabove; not strictly 100% of the barcode units need to be connected. In some embodiments, at most 30%, 20%, 10%, 5% or less of capture barcode units are not connected to any positioning barcode unit.

In some embodiments, the distribution of contact points is homogeneous in the population of barcode units. In some embodiments, the average number of contact points is similar or equal among the population of barcode units.

In some embodiments, the assembly of the population of capture barcode units with the population of positioning barcode units increases the chances of forming a contact point.

In some embodiments, the capture oligonucleotide and positioning oligonucleotide hybridize.

In a preferred embodiment, the hybridization occurs at a contact point, as defined herein. In some embodiments, at least 1%, 2%, 3%, 4%, 5%, 10%, 20%, 30%, 40%, 50%, 60%, 70%, 80%, 90%, or 100% of contact points are hybridized. In some embodiments, about 1%, 2%, 3%, 4%, 5%, 10%, 20%, 30%, 40%, 50%, 60%, 70%, 80%, 90%, or 100% of contact points are hybridized.

Thus, in a preferred embodiment, each capture barcode unit forms at least one, preferably at least 2, more preferably at least 3 hybridized contact points with distinct positioning barcode units.

In one embodiment, the capture sequence, or a fragment thereof, of the capture oligonucleotide hybridizes with the first connection sequence, or a fragment thereof, of the positioning oligonucleotide.

In another embodiment, the second connection sequence, or a fragment thereof, of the capture oligonucleotide hybridizes with the first connection sequence, or a fragment thereof, of the positioning oligonucleotide.

In some embodiments, the capture oligonucleotides cannot hybridize with the positioning oligonucleotides outside of the first connection sequence and the capture sequence. In some embodiments, the capture oligonucleotides cannot hybridize with the positioning oligonucleotides outside of the first and second connection sequences. In some embodiments, the capture oligonucleotides cannot hybridize with the positioning oligonucleotides outside of the capture sequence and the first and second connection sequences.

In some embodiments, the hybridization is stable. In some embodiments, a capture oligonucleotide hybridized with a positioning oligonucleotide does not de-hybridize. In some embodiments, a capture oligonucleotide hybridized with a positioning oligonucleotide does not de-hybridize spontaneously.

In some embodiments, hybridization of the populations of capture barcode units and positioning barcode units stabilizes the assembly of the populations of capture barcode units and positioning barcode units.

The method comprises a step of extending the capture oligonucleotide and/or the positioning oligonucleotide. Means for extending an oligonucleotide will be apparent to a person skilled in the art.

In some embodiments, the extension step can be performed on one oligonucleotide, or both oligonucleotides.

In one embodiment, the capture oligonucleotide is extended on the positioning oligonucleotide. In some embodiments, the positioning oligonucleotide is used as a template for the extension of the capture oligonucleotide.

In some embodiments, the nucleic acid that results from the extension of the capture oligonucleotide comprises the barcode sequence of the capture oligonucleotide, the barcode sequence of the positioning oligonucleotide in the same strand. In some embodiments, the nucleic acid that results from the extension of the capture oligonucleotide further comprises one or more elements selected from the group comprising or consisting of a capture sequence, one or more UMI from the capture oligonucleotide, one or more UMI from the positioning oligonucleotide, one or more PCR priming sequence from the capture oligonucleotide, and one or more PCR priming sequence from the positioning oligonucleotide. In some embodiments, the nucleic acid that results from the extension of the capture oligonucleotide further comprises one or more elements selected from the group comprising or consisting of a second connection sequence, one or more UMI from the capture oligonucleotide, one or more UMI from the positioning oligonucleotide, one or more PCR priming sequence from the capture oligonucleotide, and one or more PCR priming sequence from the positioning oligonucleotide.

In some embodiments, the nucleic acid that results from the extension of the capture oligonucleotide comprises, in 5'to 3' order:

- optionally, one or more PCR priming sequence from the capture oligonucleotide;

- the barcode sequence of the capture oligonucleotide;

- optionally, one or more UMI from the capture oligonucleotide;

- the capture sequence;

- optionally, one or more UMI from the positioning oligonucleotide;

- the barcode sequence of the positioning oligonucleotide; and

- optionally, one or more PCR priming sequence from the positioning oligonucleotide.

In some embodiments, the nucleic acid that results from the extension of the capture oligonucleotide comprises, in 5' to 3' order:

- optionally, one or more PCR priming sequence from the capture oligonucleotide;

- the barcode sequence of the capture oligonucleotide;

- optionally, one or more UMI from the capture oligonucleotide;

- the second connection sequence;

- optionally, one or more UMI from the positioning oligonucleotide;

- the barcode sequence of the positioning oligonucleotide; and

- optionally, one or more PCR priming sequence from the positioning oligonucleotide.

In another embodiment, the positioning oligonucleotide is extended on the capture oligonucleotide. In some embodiments, the capture oligonucleotide is used as a template for the extension of the positioning oligonucleotide.

In some embodiments, the nucleic acid that results from the extension of the positioning oligonucleotide comprises the barcode sequence of the positioning oligonucleotide, the barcode sequence of the capture oligonucleotide. In some embodiments, the nucleic acid that results from the extension of the positioning oligonucleotide further comprises one or more elements selected from the group comprising or consisting of a first connection sequence, one or more UMI from the positioning oligonucleotide, one or more UMI from the capture oligonucleotide, one or more PCR priming sequence from the positioning oligonucleotide, and one or more PCR priming sequence from the capture oligonucleotide. In some embodiments, the nucleic acid that results from the extension of the positioning oligonucleotide further comprises one or more elements selected from the group comprising or consisting of a second connection sequence, one or more UMI from the positioning oligonucleotide, one or more UMI from the capture oligonucleotide, one or more PCR priming sequence from the positioning oligonucleotide, and one or more PCR priming sequence from the capture oligonucleotide.

In some embodiments, the nucleic acid that results from the extension of the positioning oligonucleotide comprises, in 5' to 3' order:

- optionally, one or more PCR priming sequence from the positioning oligonucleotide;

- the barcode sequence of the positioning oligonucleotide;

- optionally, one or more UMI from the positioning oligonucleotide;

- the first connection sequence;

- optionally, one or more UMI from the capture oligonucleotide;

- the barcode sequence of the capture oligonucleotide; and

- optionally, one or more PCR priming sequence from the capture oligonucleotide.

In another embodiment, both the positioning oligonucleotide and the capture oligonucleotide are extended. In some embodiments, both the positioning oligonucleotide and the capture oligonucleotide are used as a template for each other's extension. In some embodiments, the nucleic acid that results from the extension of both the positioning oligonucleotide and the capture oligonucleotide may comprise any configuration cited hereinabove.

It will be apparent to the person skilled in the art that, after extension, the sequences corresponding to the ones comprised on the template sequence are the complementary sequence on the nucleic acid formed after extension.

It is to be understood that forming a nucleic acid that comprise barcode sequences of both a capture oligonucleotide and a positioning oligonucleotide is important for retrieving information regarding which capture oligonucleotide interacted with which positioning oligonucleotides, and therefore reconstituting neighborhood relationships between the barcode units.

The present further relates to a method for determining the spatial location of a plurality of analytes in a biological sample comprising thereof.

It is to be understood that the determination of the spatial location of the analytes relies on both (i) the method for determining the spatial location of a population of capture barcode units according to the invention, and (ii) the ability of the capture barcode units to bind to the analytes.

It is to be understood that obtaining the relative or absolute position of a specific capture barcode unit enables the deduction of the relative or absolute position of the analytes bound or otherwise associated with this barcode.

Thus, in some embodiments, the method for determining the spatial location of a plurality of analytes in a biological sample comprising thereof comprises the steps of:

(i) contacting the populations of capture barcode units and positioning barcode units of the composition according to the invention with the biological sample;

(ii) determining the spatial location of the capture barcode units by using the method according to the invention;

(iii) detecting the capture barcode unit to which each analyte is bound; and

(iv) deducing the spatial location of the analytes.

In an alternative embodiment, the method for determining the spatial location of a plurality of analytes in a biological sample comprising thereof comprises the steps of:

(i) contacting the populations of capture barcode units and positioning barcode units of the composition according to the invention with the biological sample; and

(ii) determining the spatial location of the capture barcode units by using the method according to the invention;

(iii) detecting the capture barcode unit to which each analyte is bound; and

(iv) deducing the spatial location of the analytes.

In some embodiments, the method for determining the spatial location of a plurality of analytes in a biological sample comprising thereof comprises a step of contacting the populations of capture and positioning barcode units with the biological sample, wherein this step is performed prior to the step of amplifying the plurality of nucleic acid sequences resulting from the extension of hybridized capture and positioning oligonucleotides.

In a preferred embodiment, the biological sample is lysed as described herein, in order to release the analyte.

In some embodiments, the method for determining the spatial location of a plurality of analytes in a biological sample thereof comprises a step of capturing the plurality of analytes from the biological sample by binding the plurality of analytes to the capture sequence of a plurality of capture barcode units, wherein this step is performed prior to the step of amplifying the plurality of nucleic acid sequences resulting from the extension of hybridized capture and positioning oligonucleotides, wherein this step is performed after the step of hybridizing at least one connection sequence on a positioning oligonucleotide with at least one complementary sequence on a capture oligonucleotide.

In some embodiments, the analyte is bound to the capture sequence. In some embodiments, the analyte hybridizes with the capture sequence. Illustratively, but not imitatively, a poly(dT) capture sequence may hybridize with the poly(A) tail of a mRNA. In a preferred embodiment, no more than one analyte binds to one capture oligonucleotide.

In some embodiments, the method for determining the spatial location of a plurality of analytes in a biological sample comprising thereof comprises a step of extending the capture sequence on the sequence of the plurality of analytes, thereby obtaining a plurality of nucleic acids comprising the barcode sequence of a capture barcode unit and the sequence of the analyte, wherein this step is performed prior to the step of amplifying the plurality of nucleic acid sequences resulting from the extension of hybridized capture and positioning oligonucleotides, wherein this step is performed after the step of hybridizing at least one connection sequence on a positioning oligonucleotide with at least one complementary sequence on a capture oligonucleotide.

In some embodiments, the method for determining the spatial location of a plurality of analytes in a biological sample comprising thereof comprises a step of amplifying the plurality of nucleic acids comprising the barcode sequence of a capture barcode unit and the sequence of the analyte, wherein this step is performed after to the step of extending hybridized capture and positioning oligonucleotides.

In some embodiments, the method for determining the spatial location of a plurality of analytes in a biological sample comprising thereof comprises a step of sequencing the plurality of nucleic acid sequences, or the plurality of amplicons, comprising the barcode sequence of a capture barcode unit and the sequence of the analyte.

In some embodiments, the method for determining the spatial location of a plurality of analytes in a biological sample comprising thereof comprises a step of deducing the relative position of each analyte, and optionally, deducing the absolute position of each analyte, wherein this step is performed after to the step of deducing the relative position of each capture barcode unit and/or positioning barcode unit.

In some embodiments, the method for determining the spatial location of a plurality of analytes in a biological sample comprising thereof, comprises the steps of:

(i) contacting the population of capture barcode units with the population of positioning barcode units from the composition according to the invention;

(ii) forming a plurality of contact points between the populations of capture and positioning barcode units, wherein each contact point involves one capture oligonucleotide and one positioning oligonucleotide;

(iii) at the plurality of contact points formed at step (ii), hybridizing at least one connection sequence with at least one complementary sequence on the capture oligonucleotide, thereby obtaining a plurality of hybridized oligonucleotides;

(iv) extending the plurality of hybridized oligonucleotides obtained at step (iii), thereby obtaining a plurality of nucleic acid sequences comprising the barcode sequence of a capture barcode unit, and the barcode sequence of a positioning barcode unit;

(v) optionally, amplifying the plurality of nucleic acid sequences obtained at step (iv), thereby obtaining a plurality of amplicons;

(vi) sequencing the plurality of nucleic acid sequences obtained at step (iv) or the plurality of amplicons obtained at step (v);

(vii) deducing the neighborhood relationships between the capture barcode units and the positioning barcode units;

(viii) deducing the relative position of each capture barcode unit and/or positioning barcode unit; and

(ix) optionally, deducing the absolute position of each capture barcode unit and/or positioning barcode unit;

and further comprising the steps of:

a) at any step before step (v), contacting the populations of capture and positioning barcode units with the biological sample;

b) at any step after step (a), and before step (v), capturing the plurality of analytes from the biological sample by binding the plurality of analytes to the capture sequence of a plurality of capture barcode units;

c) optionally, after step (b), extending the capture sequence on the sequence of the plurality of analytes, thereby obtaining a plurality of nucleic acids comprising the barcode sequence of a capture barcode unit and the sequence of the analyte;

d) optionally, after step (c), amplifying the plurality of nucleic acids, thereby obtaining a plurality of amplicons;

e) sequencing the plurality of nucleic acid sequences obtained at step (c) or the plurality of amplicons obtained at step (d);

f) at any step after step (viii), deducing the relative position of each analyte; and optionally, deducing the absolute position of each analyte.

In some embodiments, the biological sample is mapped prior to step (a) or prior to step (b). As used herein, "mapped" refers to any suitable mean known in the art to provide the topology or any other position information of the biological sample. In some embodiments, the biological sample is mapped by any technique selected from the group comprising or consisting of immunohistochemistry, immunohistofluorescence, *in situ* hybridization, affinity tagging, and the like.

It will be apparent to the person skilled in the art that mapping, or any spatial information, of the biological sample is useful for further optimizing the determination of the spatial location of the plurality of analytes. In some embodiments, the mapping information related to the biological sample is crossed with the relative or absolute position of the barcode units and/or analytes. In some embodiments, the mapping information related to the biological sample defines or helps define the borders of the analyte.

In some embodiments, at least one specific target is detected in the biological sample. In some embodiment, the at least one specific target is a protein, a nucleic acid, a lipid, and organelle, or combinations thereof. Methods to detect a specific target in a cell or a tissue are well known in the art. In some embodiments, the at least one specific target is used for improving the relative or absolute positioning of the barcode units and/or analytes (e.g., the specific target acts as a waypoint). In some embodiments, the at least one specific target defines the center of the biological sample.

In some embodiments, the biological sample is lysed as described herein above prior to step (b). In other words, in some embodiments, the method further comprises a step (a') of lysing the biological sample.

In one embodiment, barcoding of the analyte is achieved by primer template annealing of the barcode to the analyte, wherein the analyte is preferably a nucleic acid. In one embodiment, barcoding of the analyte is achieved by primer-directed extension of the barcode to the analyte, wherein the analyte is preferably a nucleic acid. In one embodiment, barcoding of the analyte is achieved by ligation of the barcode to the analyte, wherein the analyte is preferably a nucleic acid.

In some embodiments, the method for determining the spatial location of a plurality of analytes in a biological sample comprising thereof, comprises the steps of:

(i) contacting the first and second pluralities of barcode units from the composition according to the invention;

(ii) contacting the first and second pluralities of barcode units of step (i) with said biological sample;

(iii) forming a plurality of contact points between the populations of capture and positioning barcode units, wherein each contact point involves one capture oligonucleotide and one positioning oligonucleotide;

(iv) at the plurality of contact points formed at step (iii), hybridizing at least one connection sequence with at least one complementary sequence on said capture oligonucleotide, thereby obtaining a plurality of hybridized oligonucleotides;

(v) capturing said plurality of analytes from said biological sample by binding said plurality of analytes to the capture sequence of a plurality of capture barcode units;

(vi) optionally, extending said capture sequence on the analyte sequence, thereby obtaining a plurality of nucleic acids comprising the barcode sequence of a capture barcode unit and the sequence of the analyte;

(vii) extending the plurality of hybridized oligonucleotides obtained at step (iv), thereby obtaining a plurality of nucleic acids comprising the barcode sequence of a capture barcode unit and the barcode sequence of a positioning barcode unit;

(viii) optionally, amplifying the plurality of nucleic acids obtained at step (vii), and optionally the plurality of nucleic acids obtained at step (vi), thereby obtaining a plurality of amplicons;

(ix) sequencing the plurality of nucleic acid sequences obtained at step (vii), and optionally at step (vi) or the plurality of amplicons obtained at step (viii);

(x) deducing the neighborhood relationships between the capture barcode units and the positioning barcode units;

(xi) deducing the relative position of each analyte; and

(xii) optionally, deducing the absolute position of each analyte.

In some embodiments, the method for determining the spatial location of a plurality of analytes in a biological sample comprising thereof, comprises the steps of:

(i) contacting the first and second pluralities of barcode units from the composition according to the invention;

(ii) forming a plurality of contact points between the populations of capture and positioning barcode units, wherein each contact point involves one capture oligonucleotide and one positioning oligonucleotide;

(iii) at the plurality of contact points formed at step (iii), hybridizing at least one connection sequence with at least one complementary sequence on said capture oligonucleotide, thereby obtaining a plurality of hybridized oligonucleotides;

(iv) contacting the first and second pluralities of barcode units of step with said biological sample;

(v) capturing said plurality of analytes from said biological sample by binding said plurality of analytes to the capture sequence of a plurality of capture barcode units;

(vi) optionally, extending said capture sequence on the analyte sequence, thereby obtaining a plurality of nucleic acids comprising the barcode sequence of a capture barcode unit and the sequence of the analyte;

(vii) extending the plurality of hybridized oligonucleotides obtained at step (iii), thereby obtaining a plurality of nucleic acids comprising the barcode sequence of a capture barcode unit and the barcode sequence of a positioning barcode unit;

(viii) optionally, amplifying the plurality of nucleic acids obtained at step (vii), and optionally the plurality of nucleic acids obtained at step (vi), thereby obtaining a plurality of amplicons;

(ix) sequencing the plurality of nucleic acid sequences obtained at step (vii), and optionally at step (vi) or the plurality of amplicons obtained at step (viii);

(x) deducing the neighborhood relationships between the capture barcode units and the positioning barcode units;

(xi) deducing the relative position of each analyte; and

(xii) optionally, deducing the absolute position of each biological

In some embodiments, the method for determining the spatial location of a plurality of analytes in a biological sample comprising thereof, comprises the steps of:

(i) contacting the first and second pluralities of barcode units from the composition according to the invention;

(ii) forming a plurality of contact points between the populations of capture and positioning barcode units, wherein each contact point involves one capture oligonucleotide and one positioning oligonucleotide;

(iii) at the plurality of contact points formed at step (iii), hybridizing at least one connection sequence with at least one complementary sequence on said capture oligonucleotide, thereby obtaining a plurality of hybridized oligonucleotides;

(iv) extending the plurality of hybridized oligonucleotides obtained at step (iii), thereby obtaining a plurality of nucleic acids comprising the barcode sequence of a capture barcode unit and the barcode sequence of a positioning barcode unit;

(v) contacting the first and second pluralities of barcode units of step with said biological sample;

(vi) capturing said plurality of analytes from said biological sample by binding said plurality of analytes to the capture sequence of a plurality of capture barcode units;

(vii) optionally, extending said capture sequence on the analyte sequence, thereby obtaining a plurality of nucleic acids comprising the barcode sequence of a capture barcode unit and the sequence of the analyte;

(viii) optionally, amplifying the plurality of nucleic acids obtained at step (iv), and optionally the plurality of nucleic acids obtained at step (vii), thereby obtaining a plurality of amplicons;

(ix) sequencing the plurality of nucleic acid sequences obtained at step (iv), and optionally at step (vii) or the plurality of amplicons obtained at step (viii);

(x) deducing the neighborhood relationships between the capture barcode units and the positioning barcode units;

(xi) deducing the relative position of each analyte; and

(xii) optionally, deducing the absolute position of each analyte.

In some embodiments, the capture oligonucleotide bound to the analyte is extended. In some embodiments, the extension of the capture oligonucleotide bound to the analyte produces one or two nucleic acid molecules.

In some embodiments, the nucleic acid that results from the extension of the capture oligonucleotide bound to the analyte comprises the barcode sequence of the capture oligonucleotide, the capture sequence, and the sequence of the analyte. In some embodiments, the nucleic acid that results from the extension of the capture oligonucleotide bound to the analyte further comprises one or more UMI and one or more primer sequence.

In some embodiments, the nucleic acid that results from the extension of the capture oligonucleotide bound to the analyte comprises, in 5'to 3' order:

- optionally, one or more PCR priming sequence from the capture oligonucleotide;

- the barcode sequence of the capture oligonucleotide;

- optionally, one or more UMI from the capture oligonucleotide;

- the capture sequence; and

- the sequence of the analyte.

It will be apparent to the person skilled in the art that, after extension, the sequence of the analyte is one the form of complementary sequence on the nucleic acid formed after extension.

In some embodiments, the methods further comprise the step of forming at least one, preferably 2, complementary DNA from the nucleic acids formed by the extension of the hybridized capture and positioning oligonucleotides, and/or the nucleic acids formed by the extension of the capture oligonucleotides bound to analytes.

In a preferred embodiment, the nucleic acids formed by the extension of the hybridized capture and positioning oligonucleotides, and/or the nucleic acids formed by the extension of the capture oligonucleotides bound to analytes, are amplified.

In some embodiments, the amplification of the nucleic acids formed by the extension of the hybridized capture and positioning oligonucleotides forms "positioning amplicons".

In some embodiments, the amplification of the nucleic acids formed by the extension of the capture oligonucleotides bound to analytes or ligation of capture oligonucleotide and analyte sequence forms "analyte amplicons".

Methods to amplify a nucleic acid are well known to the person skilled in the art and include, e.g., PCR. As used herein, "amplicon" refers to the nucleic acid resulting from the amplification.

In some embodiments, the amplification is a primer-dependent amplification. Thus, in some embodiments, the amplification starts from the PCR priming sequence on the capture oligonucleotides and/or positioning oligonucleotides.

In some embodiments, the amplicons have a fixed or variable length. In some embodiments, the amplicons have a monodisperse or polydisperse length. Within the scope of the present invention, "monodisperse length" and "fixed length" are used interchangeably. Within the scope of the present invention, "polydisperse length" and "variable length" are used interchangeably. It is to be understood that the length of the capture-analyte conjugate is variable as the length of mRNA from different genes is naturally variable.

In some embodiments, the positioning amplicons have a fixed or variable size. In a preferred embodiment, positioning amplicons have a fixed size. Indeed, it is to be understood that as the sequences of the capture oligo and the positioning oligo are predetermined, it is possible to design for a desired length of the extended oligonucleotide or amplicon.

In some embodiments, the analyte amplicons have a variable or fixed size. In a preferred embodiment, analyte amplicons have a variable size. It will be apparent to the person skilled in the art that the source of length variability is that the length of the sequence of the analyte may vary greatly from one analyte to another.

In one embodiment, when the PCR priming sequence on capture oligonucleotides and the PCR priming sequence on positioning oligonucleotides are identical, one amplification is performed.

In some embodiments, the positioning amplicons and the capture amplicons are separated or isolated based on their size. In some embodiments, the positioning amplicons and the capture amplicons are separated by size exclusion methods known in the art (e.g., electrophoresis or the like).

In another embodiment, when the PCR priming sequence on capture oligonucleotides and the PCR priming sequence on positioning oligonucleotides are distinct, two amplifications are performed, typically, 2 distinct sets of primers are used.

In certain embodiments, the set of primers used comprises:

- a common forward primer (e.g., the primer for the capture oligonucleotide);

- a first reverse primer (e.g., the primer for the positioning oligonucleotide); and

- a second reverse primer (e.g., primers for the analyte).

As sequences of analytes are naturally variable, in some embodiments, universal PCR priming sequences are added to all barcoded analytes. This can be done for example by second strand synthesis with synthetic oligonucleotides having degenerative sequence at 3' and universal common sequence at 5'. In another, less preferred embodiment, a part of cDNA sequence of all or part of genes of interest is used as a collection of selective PCR primers of barcoded analytes.

In some embodiments, the amplicons resulting from the two distinct amplifications are separated or isolated. In some embodiments, the positioning amplicons and the capture amplicons are formed separately.

In certain embodiments, the methods according to the present invention may comprise a step of pre-amplification of the analyte.

It will be apparent to the person skilled in the art that, for the analyte captured by capture oligonucleotide, extension happens essentially from the 3' end of the capture oligo by reverse transcription, followed by addition of the cDNA sequence to the 3' end of the capture oligonucleotide. This addition can be performed by any means such as ligation.

In some embodiments, the methods further comprise the step of forming at least one, preferably 2, libraries of nucleic acids or amplicons, preferably amplicons, as defined hereinabove.

In a preferred embodiment, the methods further comprise the step of forming 2 libraries of nucleic acids or amplicons, preferably amplicons, as defined hereinabove. In some embodiments, the 2 libraries consist of:

- a first library comprising nucleic acids formed by the extension of the hybridized capture and positioning oligonucleotides, or positioning amplicons; and

- a second library comprising nucleic acids formed by the extension of the capture oligonucleotides bound to analytes, or analyte amplicons.

Within the scope of this invention, the first library is interchangeably referred to as "positioning library", and the second library is interchangeably referred to as "analyte library".

In some embodiments, the first library comprises the positioning information of capture barcode units, and the second library comprises the analyte sequence information linked to each capture barcode units.

In some embodiments, the methods further comprise the step of sequencing the libraries of nucleic acids or amplicons, preferably amplicons, as defined hereinabove. As used herein, for the sequencing step, the nucleic acids formed by the extension of the hybridized capture and positioning oligonucleotides, or positioning amplicons, as well as the nucleic acids formed by the extension of the capture oligonucleotides bound to analytes, or analyte amplicons, are interchangeably referred to as "fragments" or "reads" for the sequencing.

In a preferred embodiment, each capture barcode unit forms at least one, preferably at least 2, more preferably at least 3 sequenced contact points with distinct positioning barcode units.

Methods to sequence a nucleic acid are known to the person skilled in the art.

In one embodiment, the nucleic acids formed by the extension of the hybridized capture and positioning oligonucleotides, or positioning amplicons, and/or the nucleic acids formed by the extension of the capture oligonucleotides bound to analytes, or analyte amplicons are sequenced in their entirety, *i.e.*, over the all length of their nucleic acid sequence.

In one embodiment, the nucleic acids formed by the extension of the hybridized capture and positioning oligonucleotides, or positioning amplicons, and/or the nucleic acids formed by the extension of the capture oligonucleotides bound to analytes, or analyte amplicons are partially sequenced. In a preferred embodiment, the part that is not sequenced corresponds to the contact point and/or to the sequence hybridized between a capture sequence and an analyte.

It is to be understood that, in order to facilitate sequencing, the libraries, preferably the first and second libraries as defined hereinabove, are separated. As described above, separation can be achieved by:

- separation of amplicons having distinct sizes, wherein the positioning amplicons have a fixed size, by means of size exclusion;

- separate amplification, by means of separate amplification reactions (and, *e.g.*, use of distinct sets of primers); or

- separation of capture barcode units and positioning barcode units prior to amplification.

For further optimization of the method, constructing the library comprising the positioning information of capture barcode units requires the minimum sequencing necessary to reconstitute positioning information; however, constructing the library comprising the analyte sequence information linked to each capture barcode units requires extensive and complete sequencing, in order to maximize the amount of information obtained from the biological sample. It is thus important to control the ratio between positioning library and analyte library for sequencing both libraries simultaneously, or sequence each library in separate sequencing run at optimal depth for each library.

In one embodiment, the nucleic acids or amplicons as defined hereinabove are separated based on their length. In one embodiment, when the PCR priming sequence on capture oligonucleotides and the PCR priming sequence on positioning oligonucleotides are identical and one amplification is performed, the nucleic acids or amplicons as defined hereinabove are separated based on their length. Length-based or size-based separation of nucleic acids are routine techniques well known to the person skilled in the art.

In another embodiment, when the PCR priming sequence on capture oligonucleotides and the PCR priming sequence on positioning oligonucleotides are distinct and two distinct amplifications are performed, the positioning amplicons and the analyte amplicons are formed separately. Thus, in some embodiments, the methods further comprise the substep of:

- generating positioning amplicons, as described herein;

- collecting and/or isolating the positioning amplicons;

- generating analyte amplicons, as described herein; and

- collecting and/or isolating the analyte amplicons.

or

- generating analyte amplicons, as described herein;

- collecting and/or isolating the analyte amplicons;

- generating positioning amplicons, as described herein; and

- collecting and/or isolating the positioning amplicons.

In another embodiment, the nucleic acids formed by the extension of the hybridized capture and positioning oligonucleotides, and the nucleic acids formed by the extension of the capture oligonucleotides bound to analytes, are separated prior to amplification. In certain embodiments, the nucleic acids formed by the extension of the hybridized capture and positioning oligonucleotides, and the nucleic acids formed by the extension of the capture oligonucleotides bound to analytes, are separated prior to sequencing.

It may be practical, for separation prior to libraries construction, to use magnetic force, centrifugation, sedimentation, size exclusion, or the like. In some embodiments, the two populations of barcode units are separated by magnetic force, wherein one type of barcode unit is magnetic and the other is non-magnetic. In some embodiments, the two populations of barcode units are separated by size exclusion, wherein capture barcode units and positioning barcode units have distinct sizes. In some embodiments, the two populations of barcode units are separated by centrifugation or sedimentation, capture barcode units and positioning barcode units have distinct densities.

Therefore, one PCR from separated capture barcode units generates the analyte library, and the other PCR from separated positioning barcode units generates the positioning library.

In some embodiments, the capture barcode units and/or positioning barcode units comprise a mean for their separation and/or isolation. In some embodiments, one type of barcode unit is magnetic and the other is non-magnetic, and the method further comprises at least one means for separating magnetic barcode units.

It will be apparent to the person skilled in art that the separation prior to libraries construction is useful for maximizing the collection of information from the biological sample while collecting the minimal required positioning information. In other words, the separation prior to libraries construction enables a higher degree of freedom or flexibility to adjust the depth of sequencing independently for each library.

It will be understood that the present invention aims at obtaining information regarding the spatial location of barcode units in an assembly of 2 types of barcode units, and/or the spatial location of analytes in a biological sample. The spatial location may be relative (*e.g.*, barcode units relative to each other barcode unit and/or analytes relative to each other capture barcode unit) or absolute (*e.g.*, barcode units relative to a known point of reference and/or analytes relative to a known point of reference).

In some embodiments, the relative position of a capture barcode unit and/or a positioning barcode unit is defined with respect to each other barcode unit.

In some embodiments, the absolute position of a capture barcode unit and/or a positioning barcode unit is defined with respect to the absolute position of a known point of reference. In one embodiment, the known point of reference is the center point of the assembly of barcode units, preferably the geometrical center point of the assembly of barcode units. In another embodiment, the known point of reference belongs to the boundary of the assembly of barcode units.

In some embodiments, the relative position of an analyte is defined with respect to each capture barcode unit.

In some embodiments, the absolute position of an analyte is defined with respect to the absolute position of a known point of reference. In one embodiment, the known point of reference is the center point of the assembly of barcode units, preferably the geometrical center point of the assembly of barcode units. In another embodiment, the known point of reference belongs to the boundary of the assembly of barcode units. In an alternative, less preferred embodiment, the absolute position of an analyte is defined with respect to the absolute position of a biological point of reference on the biological sample, wherein the biological point of reference is a protein, a complex of proteins, a nucleic acid, an organelle or the like, preferably the biological point of reference is labelled or tagged by any method known in the art. In some embodiments, the absolute position of an analyte reflects the position of the analyte in the biological sample, *e.g.*, the position of the analyte in a definite area of the biological sample, or in a definite cell of the biological sample.

It is to be understood that determining the position of barcode units relative to each other and/or relative to a known point of reference requires obtaining and recapitulating the neighborhood relationships between barcode units, *i.e.,* representing the network or array of connections between the capture barcode units and the positioning barcode units. As used herein, "connections" means hybridized contact points, preferably sequenced contact points. In some embodiments, the connections between the capture barcode units and the positioning barcode units are represented in the form of at least one graph, preferably one graph. In a preferred embodiment, all barcode units that have formed at least one connection are comprised in the at least one graph, preferably one graph.

Therefore, in some embodiments, methods for the determination of the relative or absolute position of the barcode units, preferably capture barcode units, or analytes, comprises the use of at least one algorithm, and at least one input for the algorithm.

As used herein, the variables used in the algorithm comprise the following:

- $N_c$ : number of capture barcode units;

- $N_p$ : number of positioning barcode units;

- $N_a$ : number of analytes;

- $\rho_c$ : radius of each capture barcode unit;

- $\rho_p$ : radius of each positioning barcode unit;

- $L_c(i)$ : spatial location of the *i*-th capture barcode with respect to the center of the set of barcode units, preferably wherein the center is the geometrical center

- $L_a(m)$ : spatial location of the *m*-th analyte with respect to the center of the set of barcode units, preferably wherein the center is the geometrical center;

- $\chi(i, j)$ : number of sequenced fragments *(i.e., reads)* including both the barcode sequence of the *i*-th capture barcode unit and the barcode sequence of the *j*-th positioning barcode unit;

- *C(i, m)* : number of sequenced fragments *(i.e., reads)* including both the barcode sequence of the *i*-th capture barcode unit and the information of the sequence of the m-th analyte.

In some embodiments, $\rho_c$ and/or $\rho_p$ are expressed in $\mu$m. In some embodiments, coordinates values of $L_c(i)$ and/or $L_a(m)$ are in $\mu$m.

In some embodiments, the spatial resolution for the spatial location of the analyte is at the level of the capture barcode units. In some embodiments, if $C(i, m) \geq$ 1, it is approximated that $L_a(m) = L_c(i)$.

In some embodiments, the at least one algorithm comprises or consists of at least one graph of the interactions between capture barcode units. In some embodiments, the graph is used as input for the at least one algorithm.

In one embodiment, a graph G is used as input. In some embodiments, graph G reflects where the nodes are the capture barcode units and the positioning barcode units, and where the edges are connecting the *i*-th capture barcode unit and the *j*-th positioning barcode unit, if and only if $\chi(i, j) \geq 1$.

It will be understood that, by applying state-of-art manifold learning or graph embedding methods to the graph *G*, it is possible to estimate the relative position $L_c(i)$ for each capture barcode *i*, and then the relative position $L_a(m)$ of each captured analyte *m*.

In certain embodiments, graph $G_\chi$, a weighted version of graph G, is used as input to take into account some connection uncertainty. In some embodiments, the weighted graph $G_\chi$ reflects where the nodes are the capture barcode units and the positioning barcode units, where the edges are connecting the *i*-th capture barcode unit and the j-th positioning barcode unit if and only if $\chi(i, j) \geq 1$, and where the weight of such edges is $\chi(i,j)$.

In certain embodiments, graph $G_\psi$, a weighted version of graph G, is used as input, preferably when the capture oligonucleotides and positioning oligonucleotides comprise UMIs, in order to ignore PCR clones. In some embodiments, $\psi(i, j)$ is the number of distinct sequenced fragments (i.e., paired reads) including both the barcode sequence of the *i*-th capture barcode unit and the barcode sequence of the *j*-th positioning barcode unit, after the removal of PCR clones based on the UMI associated with the *i*-th capture barcode unit and the UMI associated with the *j*-th positioning barcode unit.

In another embodiment, graph $\acute{G}$ is used as input. In some embodiments, graph $\acute{G}$ reflects where

the nodes are restricted to the capture barcode units, and where the edges are connecting the *i*-th capture barcode unit and the *k*-th capture barcode unit if and only if there is a positioning barcode unit *j* such that $\chi(i,j) \geq 1$ and $\chi(k,j) \geq 1$.

In certain embodiments, graph $\acute{G}_\omega$ is used as input, wherein graph $\acute{G}_\omega$ is a weighted version of graph $\acute{G}$, wherein the weight of edges is $\omega(i, k) =$

$$\sum_{j=1}^{N_p} \min(\chi(i, j), \chi(k, j))$$ .

In certain embodiments, graph $\acute{G}_\theta$ is used as input, preferably when the capture oligonucleotides and positioning oligonucleotides comprise UMIs, in order to ignore PCR clones, wherein graph $\acute{G}_\theta$ is weighted version of graph G, wherein

$$\theta(i,k) = \sum_{j=1}^{N_p} \min(\psi(i, j), \psi(k, j))$$ .

In certain embodiments, graph $\acute{G}_\gamma$ is used as input, wherein graph $\acute{G}_\gamma$ is a weighted version of graph G, wherein the weight of edges is

$$\gamma(i, k) = \sum_{j=1}^{N_p} \delta_{ij}\, \delta_{kj}$$ , wherein $\delta_{ij}$ is the Kronecker delta function equal to 1 if $\chi(i, j) \geq 1$ and equal to 0 otherwise.

In some embodiments, the determination of the relative or absolute position of the barcode units comprises using at least one multidimensional scaling technique for graph embedding and manifold learning. In some embodiments, the at least one multidimensional scaling technique is selected from the group comprising or consisting of local methods (e.g., Laplacian Eigenmaps, LLE and Diffusion Maps), global methods (*e.g.*, Isomap and Landmark Isomap) or neural network-based methods (*e.g.*, DIMAL: Deep Isometric MAnifold Learning). Such methods are known in the art (see, *e.g.,* Pai et al., 2022, "Deep Isometric Maps"). In some embodiment, the at least one algorithm is any suitable algorithm known in the art. In some embodiment, the at least one algorithm is a manifold learning algorithm. In some embodiments, the at least one algorithm is ISOMAP.

In some embodiments, the use of the algorithm comprises a step of estimating geodesic distances between the nodes of the graph in order to deduce the relative spatial location of the barcode units with respect to each other, wherein a geodesic distance between two nodes is the distance of the shortest path connecting the two nodes via the edges of the graph.

As such, in some embodiments, theses distances can be described using the following distance functions:

- *D(i, j)* being the geodesic distance between the center of the *i*-th capture barcode unit and the center of the j-th positioning barcode unit; and

- *E(i, k)* being geodesic distance between the center of the *i*-th capture barcode unit and the center of the *k*-th capture barcode unit.

In some embodiments, according to triangle inequality, $E(i, k) \leq E(i, l) + E(l, k)$ and $D(i, k) \leq D(i, j) + D(j, k)$.

In some embodiments, in case of rigid and spherical barcode units:

- if $\chi(i, j) \geq 1$ then $D(i, j) \approx \rho_c + \rho_p$;

- if $\chi(i,j) \geq 1$ and $\chi(k,j) \geq 1$ then $2\rho_c \leq E(i, k) \leq 2(\rho_c + \rho_p)$;

- if the capture barcode units form a compact hexagonal mesh wherein each capture barcode unit is in physical contact with 6 other barcode units, then: if $\chi(i, j) \geq 1$ and $\chi(k, j) \geq 1$ then $E(i, k) \approx 2\rho_c$.

As used herein, the following variables are defined for describing the connections between the two populations of barcode units:

- $$e_c(j) = \sum_{i=1}^{N_c} \delta_{ij}$$ represents the number of capture barcode units having at least one sequenced contact point with the positioning barcode unit *j*;

- $$e_p(i) = \sum_{j=1}^{N_p} \delta_{ij}$$ represents the number of positioning barcode units having at least one sequenced contact point with the capture barcode unit i.

In one embodiment, if each positioning barcode unit is connected to at most N capture barcode units, then: if $e_c(j) > N$, there is probably a barcode collision at the level of the sequence barcode of the positioning barcode unit *j (i.e.,* two identical barcode sequences on the oligonucleotides of two distinct positioning barcode units) and one can decide to exclude the positioning barcode unit *j* from the graph of Interest.

In another embodiment, if each capture barcode unit is connected to at most N positioning barcode units, then: if $e_p(i) > N$, there is probably a barcode collision at the level of the sequence barcode of the capture barcode unit *i (i.e.,* two identical barcode sequences on the oligonucleotides of two distinct capture barcode units) and one can decide to exclude the capture barcode unit i from the graph of interest.

In some embodiments, $S(i, k)$ represents a pre-defined similarity score between the biological signature of the analytes captured by the *i*-th capture barcode unit and the biological signature of the analytes captured by the k-th capture barcode unit, wherein an analyte is said to be captured by the *i*-th capture barcode unit if there is at least one sequenced fragment (i.e., read) including both the barcode sequence of the *i*-th capture barcode and the information of the sequence of the analyte.

In some embodiments, the determination of the relative or absolute position of the barcode units comprises the steps of:

i. using at least one of the pre-defined graphs as defined herein, and optionally using the similarity matrix $S(.,.)$ as defined herein, to identify the connected component of the input graph(s), where a connected component in a graph is defined as a set of barcode units that are linked to each other by paths in the graph;

ii. for each connected component, estimating the geodesic distance (*i.e.,* the shortest path in the graph of interest) between the centers of any two capture barcode units belonging to the connected component;

iii. for each said connected component, deducing the spatial locations of the center of each capture barcode units belonging to the connected component, wherein the spatial location is defined with respect to the absolute location of the center of the connected component;

iv. for each said component, deducing the spatial location of the center of each analyte captured by a capture barcode unit belonging to the connected component, wherein the spatial location is defined with respect to the absolute location of the center of the connected component;

v. optionally, using the similarity matrix $S(.,.)$ and/or the shape of the boundary of each said connected component, estimate the geodesic distance between the centers of any two capture barcode units belonging to the boundary of two distinct connected components, then estimate the spatial location of the center of each analyte captured by the capture barcode units, wherein the spatial location is defined with respect to the absolute location of the center of the set of barcode units; and

vi. optionally, leverage the knowledge of the absolute location of at least 3 barcode units as spatial landmarks and/or a complementary mapping information such as a microscope image (e.g., fluorescence image) of the biological sample, in order to apply a local adjustment (*e.g.,* translation, rotation, shearing) to the estimated spatial locations.

It is to be understood that the determination of the relative or absolute position of the barcode units comprises may require, or may be facilitated by, several conditions, as detailed non-limitatively hereinbelow.

In a preferred embodiment, for a substantial proportion of the capture barcode units, each capture barcode unit is connected to at least one positioning barcode unit which is connected to at least one other capture barcode unit, and each capture barcode unit is connected to at least one analyte of the biological sample. As used herein, a "substantial proportion" means at least 50%, 60%, 70%, 80%, 90%, 95%, 99%, or more, of the total population of capture barcode units. In certain embodiments, each positioning barcode unit is in physical contact with three capture barcode units. In some embodiments, each capture barcode unit, except those at the boundary of the assembly, is in physical contact with six capture barcode units. In some embodiments, each capture barcode unit, except those at the boundary of the assembly, is in physical contact with five positioning barcode units.

Some configurations of the capture barcode units may facilitate the determination of the relative or absolute position of the barcode units. In some embodiments, all capture barcode units have the same radius, and all positioning barcode units have the same radius. In some embodiments, capture barcode units are rigid spherical units, while positioning barcode units are semi-solid units. In some embodiments, positioning barcode units are smaller than capture barcode units, for example two times smaller in diameter.

In some embodiments, the biological sample lies on a planar surface. In some embodiments, the assembly of capture barcode units is a compact hexagonal mesh.

In some embodiments, the graph $G$ of physical contacts between capture barcode units and positioning barcode units is made of only one connected component. As used herein, "connected component" refers to one ensemble of connected barcode units. In some embodiments, the graph $G$ of physical contacts between capture barcode units and positioning barcode units contains as many cycles as possible, where a cycle is defined as a closed path in the graph (*i.e.*, which starts and ends at the same barcode unit) and which contains at least 3 distinct barcode units.

In some embodiments, the graph $G$ of physical contacts between capture barcode units and positioning barcode units is homogeneous. In some embodiments, the graph $G$ of physical contacts between capture barcode units and positioning barcode units is biconnected. In some embodiments, the graph $G$ of physical contacts between capture barcode units and positioning barcode units is multicyclic.

In some embodiments, 1, 2, 3 or 4 distinct connected components, are formed in a graph of interest. In a preferred embodiment, only one connected component, is formed in a graph of interest.

In some embodiments, when more than one connected component, is formed in a graph of interest, the topology is reconstituted by other means.

Some configurations of the capture barcode units may further facilitate the determination of the relative or absolute position of the barcode units, but are optional and not strictly necessary for the method of the invention.

In some embodiments, the absolute spatial location of the center of set of barcode units is known, with respect to the center, or any anchoring point, of the biological sample.

In some embodiments, the absolute spatial location of at least two barcode units (capture or positioning barcode units) is known, where the absolute spatial location is defined with respect to the absolute spatial location of the center of the assembly of barcode units.

In some embodiments, the absolute spatial location of at least one barcode unit belonging to each connected component of the graph $G$ is known, where the absolute spatial location is defined with respect to the absolute spatial location of the center of the assembly of barcode units.

In some embodiments, some similarity scores are known between the biological signature of the analytes captured by distinct capture barcode units and/or in distinct connected components of a graph of interest.

The present invention further relates to a kit comprising the composition according to the invention, and instructions for use.

The present invention further relates to a kit comprising the substrate according to the invention, and instructions for use.

**BRIEF DESCRIPTION OF THE DRAWINGS**

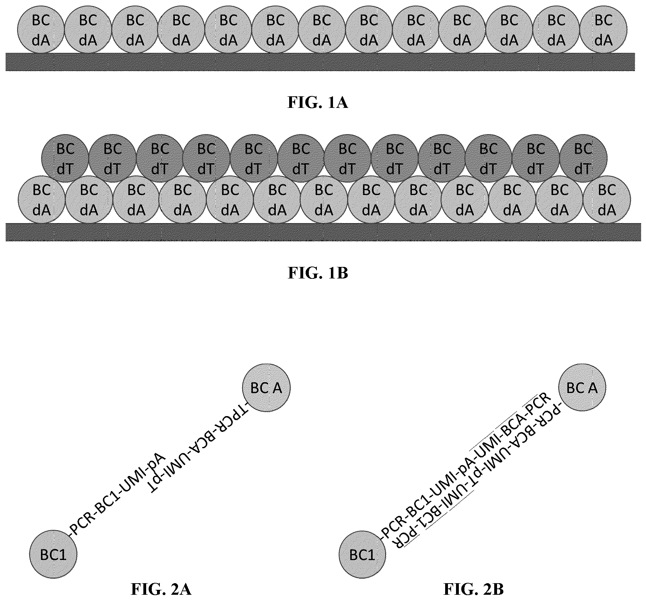

**Figure 1A-1B** is a set of schematics showing an example of assembly of capture and positioning barcode units. **Fig. 1A** shows a monolayer of positioning barcoded beads (BC) comprising a poly(dA) sequence as connection sequence; **Fig. 1B** shows the addition of a monolayer of capture barcode units comprising a poly(dT) sequence as capture sequence.

**Figure 2A-2B** is a set of schematics showing an example of hybridization (**Fig. 2A**) and extension (**Fig. 2B**) of a positioning oligonucleotide (BC1) with a capture oligonucleotide (BC A).

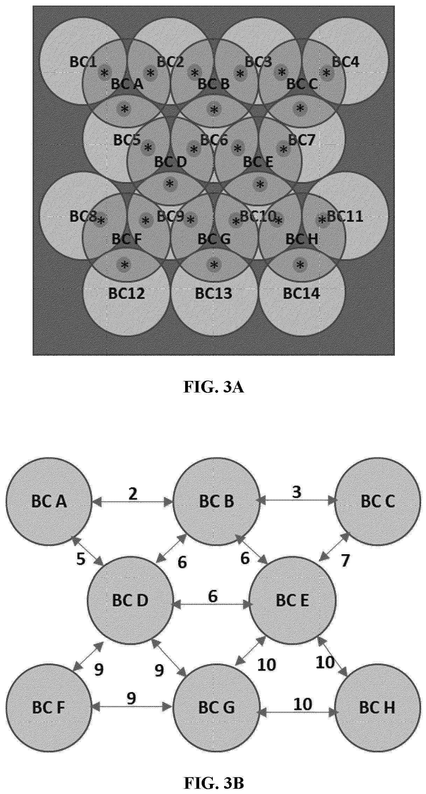

**Figure 3A-3B** is a set of schematics showing an example of packing of positioning barcode units (BC1, BC2, BC3, etc.) with capture barcode units (BC A, BC B, BC C, etc.), wherein contact surface area indicated by an asterisk (*) (**Fig. 3A**) and of reconstruction of relative positions (**Fig. 3B**). The numbers on the edges represent the identification number of a given positioning barcode unit bound to the capture barcode unit.

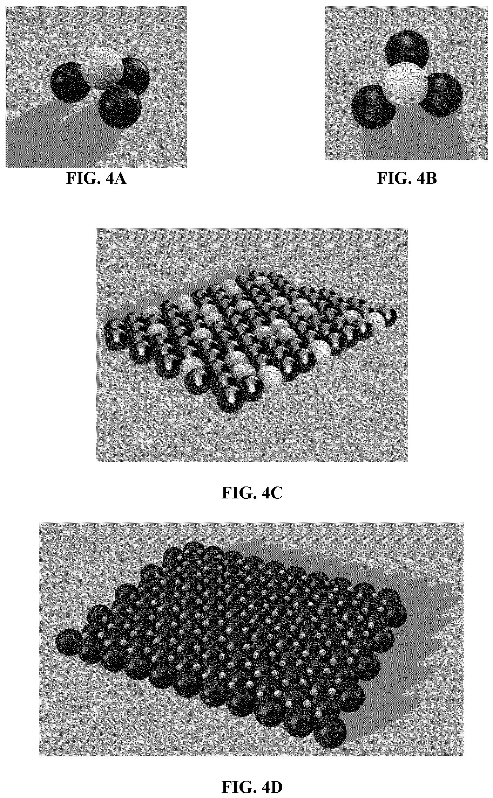

**Figure 4A-4D** is a set of schematics showing examples of packing the barcode units. **Fig. 4A** and **4B** show one barcode unit of the first type binding to 3 barcode units of the second type. **Fig. 4C** and **4D** show close packing of the barcode units of the two types, when the two types of barcode units have the same size (**Fig. 4C**) or different sizes (**Fig. 4D**). White beads are capture barcode units; black beads are positioning barcode units.

**EXAMPLES**

The present invention is further illustrated by the following examples.

Example 1:

**Figure 1A-1B** shows an example of positioning barcode units, comprising a poly(A) sequence as a first connection sequence, and deposited on a support and forming a layer **(Fig. 1A)** completed with a second layer of capture barcode units, comprising a poly(T) sequence as capture sequence (**Fig. 1B**).
Such support comprising both layers can be used for spatial omics applications on a biological sample. Indeed, hybridization between a positioning barcode unit and a capture barcode unit (**Fig. 2A**) followed by extension of the oligonucleotides (**Fig. 2B**) allows for reconstruction of connection between barcode units of the 2 populations by the formation and sequencing of nucleic acid sequences comprising both the barcode sequences of both positioning and capture barcode units.
Various types of assemblies can be performed (see **Figure 4**) depending on the density of size of the barcode units, for example.

Example 2:

**Figure 3** shows an example of determination of the spatial location of capture barcode units. In a complex mix comprising both populations of barcode units (**Fig. 3A**), the neighborhood relationships between the capture barcode units are defined by their connections with the positioning barcode units (**Fig. 3A**).

**Claims**

1. A composition comprising:

   - a population of capture barcode units, wherein each capture barcode unit comprises a population of capture oligonucleotides, wherein each capture oligonucleotide comprises a barcode sequence, and a capture sequence capable of binding an analyte from a biological sample;
   - a population of positioning barcode units, wherein each positioning barcode unit comprises a population of positioning oligonucleotides, wherein each positioning oligonucleotide comprises a barcode sequence, and a first connection sequence that can hybridize on a capture oligonucleotide;

   wherein each barcode unit comprises a distinct barcode sequence, and all oligonucleotides on a same barcode unit comprise the same barcode sequence.

2. The composition according to claim **1**, wherein said capture sequence cannot hybridize with said capture sequence, and wherein said first connection sequence cannot hybridize with said first connection sequence.

3. The composition according to claim **1** or **2**, wherein said first connection sequence of the positioning oligonucleotides can hybridize on said capture sequence of the capture oligonucleotides.

4. The composition according to claims **1** to **3**, wherein said capture oligonucleotides comprise a second connection sequence, wherein said second connection sequence is distinct from said barcode sequence and said capture sequence, and wherein said first connection sequence of the positioning oligonucleotides can hybridize on said second connection sequence of the capture oligonucleotides.

5. The composition according to any one of claims **1** to **4**, wherein said capture sequence is identical for all capture barcode units, and said first connection sequence is identical for all positioning barcode units.

6. The composition according to any one of claims **1** to **5**, wherein said capture oligonucleotides and/or said positioning oligonucleotides further comprise at least one PCR priming sequence.

7. The composition according any one of claims **1** to **6**, wherein each oligonucleotide from said population of capture oligonucleotides and/or said population of positioning oligonucleotides further comprise at least one unique molecular identifier (UMI).

8. The composition according to any one of claims **1** to 7, wherein said capture oligonucleotides comprise, in the 5' to 3' order: optionally at least one linker, optionally at least one PCR priming sequence, said barcode sequence, optionally at least one UMI, and said capture sequence; and wherein said positioning oligonucleotides comprise, in the 5' to 3' order: optionally at least one linker, optionally at least one PCR priming sequence, said barcode sequence, and said first connection sequence.

9. The composition according any one of claims **1** to **8**, wherein said barcode units are synthetic barcode units.

10. The composition according any one of claims **1** to **9**, wherein said barcode units are beads or DNA nanoballs.

11. The composition according any one of claims **1** to **10**, wherein said population of capture barcode units and said population of positioning barcode units are assembled on a substrate.

12. The composition according any one of claim **1** to **11**, wherein said capture barcode units are in contact with at least one, preferably at least 2, more preferably at least 3 said positioning barcode units.

13. A method for determining the spatial location of a population of capture barcode units, comprising the steps of:

    (i) contacting the population of capture barcode units with the population of positioning barcode units from the composition according to any one of claims **1** to **12**;
    (ii) forming a plurality of contact points between the populations of capture and positioning barcode units, wherein each contact point involves one capture oligonucleotide and one positioning oligonucleotide;
    (iii) at the plurality of contact points formed at step (ii), hybridizing at least one connection sequence with at least one complementary sequence on said capture oligonucleotide, thereby obtaining a plurality of hybridized oligonucleotides;
    (iv) extending the plurality of hybridized oligonucleotides obtained at step (iii), thereby obtaining a plurality of nucleic acid sequences comprising the barcode sequence of a capture barcode unit, and the barcode sequence of a positioning barcode unit;
    (v) optionally, amplifying the plurality of nucleic acid sequences obtained at step (iv), thereby obtaining a plurality of amplicons;
    (vi) sequencing the plurality of nucleic acid sequences obtained at step (iv) or the plurality of amplicons obtained at step (v);
    (vii) deducing the neighborhood relationships between the capture barcode units and the positioning barcode units;
    (viii) deducing the relative position of each capture barcode unit and/or positioning barcode unit; and
    (ix) optionally, deducing the absolute position of each capture barcode unit and/or positioning barcode unit.

14. A method for determining the spatial location of a plurality of analytes in a biological sample comprising thereof, comprising the steps of:

    (i) contacting the population of capture barcode units with the population of positioning barcode units from the composition according to any one of claims **1** to **12**;
    (ii) forming a plurality of contact points between the populations of capture and positioning barcode units, wherein each contact point involves one capture oligonucleotide and one positioning oligonucleotide;
    (iii) at the plurality of contact points formed at step (ii), hybridizing at least one connection sequence with at least one complementary sequence on said capture oligonucleotide, thereby obtaining a plurality of hybridized oligonucleotides;
    (iv) extending the plurality of hybridized oligonucleotides obtained at step (iii), thereby obtaining a plurality of nucleic acid sequences comprising the barcode sequence of a capture barcode unit, and the barcode sequence of a positioning barcode unit;
    (v) optionally, amplifying the plurality of nucleic acid sequences obtained at step (iv), thereby obtaining a plurality of amplicons;
    (vi) sequencing the plurality of nucleic acid sequences obtained at step (iv) or the plurality of amplicons obtained at step (v);
    (vii) deducing the neighborhood relationships between the capture barcode units and the positioning barcode units;
    (viii) deducing the relative position of each capture barcode unit and/or positioning barcode unit; and
    (ix) optionally, deducing the absolute position of each capture barcode unit and/or positioning barcode unit;

and further comprising the steps of:

    a) at any step before step (v), contacting the populations of capture and positioning barcode units with said biological sample;
    b) at any step after step (a), and before step (v), capturing said plurality of analytes from said biological sample by binding said plurality of analytes to the capture sequence of a plurality of capture barcode units;
    c) optionally, after step (b), extending said capture sequence on the sequence of said plurality of analytes, thereby obtaining a plurality of nucleic acids comprising the barcode sequence of a capture barcode unit and the sequence of the analyte;
    d) optionally, after step (c), amplifying the plurality of nucleic acids, thereby obtaining a plurality of amplicons;
    e) sequencing the plurality of nucleic acid sequences obtained at step (c) or the plurality of amplicons obtained at step (d);
    f) at any step after step (viii), deducing the relative position of each analyte; and optionally, deducing the absolute position of each analyte in said biological sample.

**15.** The method according to claim **14,** wherein said biological sample is selected from the group comprising or consisting of a tissue, a tissue section, a monolayer of cells, multiple layers of cells, an organoid, a syncytium or a single cell.

FIG. 1A

FIG. 1B

FIG. 2A

FIG. 2B

FIG. 3A

FIG. 3B

**FIG. 4A**

**FIG. 4B**

**FIG. 4C**

**FIG. 4D**

Europäisches
Patentamt

European
Patent Office

Office européen
des brevets

# EUROPEAN SEARCH REPORT

**Application Number**

**EP 23 30 6160**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/025446 A1 (SHAH PREYAS [US]) 27 January 2022 (2022-01-27) * paragraphs [0287]-[0288], [0333], [0344], [0339], [0378]-[0379], [0395], [0416], [0518], [0973]; claims 44, 55, 64; figures 6, 8, 9, 10 * | 1,3,5-15 | INV. C12Q1/6837 C12Q1/6841 |
| X | US 2021/155982 A1 (YIN YIFENG [US] ET AL) 27 May 2021 (2021-05-27) * paragraphs [0315]-[0318], [0398]-[0406]; figures 6, 40 * | 1,8 | |
| X | US 2022/017952 A1 (FAN HEI MUN CHRISTINA [US] ET AL) 20 January 2022 (2022-01-20) * paragraphs [0023]-[0024], [0028], [0037], [0040], [0046], [0051]-[0052], [0061]-[0062], [0065], [0066]-[0067], [0071]; figures 1A, 3A, 5 * | 1-4,6-11 | |

**TECHNICAL FIELDS SEARCHED (IPC)**

C12Q

The present search report has been drawn up for all claims

| Place of search | Date of completion of the search | Examiner |
|---|---|---|
| Munich | 18 December 2023 | Celler, Jakub |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 30 6160

This annex lists the patent family members relating to the patent documents cited in the above-mentioned European search report.
The members are as contained in the European Patent Office EDP file on
The European Patent Office is in no way liable for these particulars which are merely given for the purpose of information.

18-12-2023

| Patent document cited in search report | Publication date | Patent family member(s) | | Publication date |
|---|---|---|---|---|
| US 2022025446 A1 | 27-01-2022 | CN | 113439124 A | 24-09-2021 |
| | | CN | 113767176 A | 07-12-2021 |
| | | CN | 113767177 A | 07-12-2021 |
| | | EP | 3894585 A2 | 20-10-2021 |
| | | EP | 3894586 A2 | 20-10-2021 |
| | | EP | 3894587 A1 | 20-10-2021 |
| | | EP | 3894588 A1 | 20-10-2021 |
| | | EP | 3894589 A2 | 20-10-2021 |
| | | EP | 3894590 A2 | 20-10-2021 |
| | | EP | 3894591 A2 | 20-10-2021 |
| | | EP | 3894592 A2 | 20-10-2021 |
| | | SG | 11202105824R A | 29-06-2021 |
| | | US | 2020277663 A1 | 03-09-2020 |
| | | US | 2020277664 A1 | 03-09-2020 |
| | | US | 2022025446 A1 | 27-01-2022 |
| | | US | 2022025447 A1 | 27-01-2022 |
| | | US | 2022033888 A1 | 03-02-2022 |
| | | US | 2022049294 A1 | 17-02-2022 |
| | | US | 2022064630 A1 | 03-03-2022 |
| | | US | 2022112486 A1 | 14-04-2022 |
| | | US | 2022241780 A1 | 04-08-2022 |
| | | US | 2022290217 A1 | 15-09-2022 |
| | | WO | 2020123301 A2 | 18-06-2020 |
| | | WO | 2020123305 A2 | 18-06-2020 |
| | | WO | 2020123309 A1 | 18-06-2020 |
| | | WO | 2020123311 A2 | 18-06-2020 |
| | | WO | 2020123316 A2 | 18-06-2020 |
| | | WO | 2020123317 A2 | 18-06-2020 |
| | | WO | 2020123318 A1 | 18-06-2020 |
| | | WO | 2020123319 A2 | 18-06-2020 |
| | | WO | 2020123320 A2 | 18-06-2020 |
| US 2021155982 A1 | 27-05-2021 | AU | 2020388573 A1 | 23-06-2022 |
| | | CA | 3158888 A1 | 27-05-2021 |
| | | CN | 115210760 A | 18-10-2022 |
| | | CN | 117078725 A | 17-11-2023 |
| | | EP | 4062373 A1 | 28-09-2022 |
| | | US | 2021155982 A1 | 27-05-2021 |
| | | WO | 2021102039 A1 | 27-05-2021 |
| US 2022017952 A1 | 20-01-2022 | CN | 116209796 A | 02-06-2023 |
| | | EP | 4182328 A1 | 24-05-2023 |
| | | US | 2022017952 A1 | 20-01-2022 |
| | | US | 2023212659 A1 | 06-07-2023 |
| | | WO | 2022015925 A1 | 20-01-2022 |

EPO FORM P0459

For more details about this annex : see Official Journal of the European Patent Office, No. 12/82

## REFERENCES CITED IN THE DESCRIPTION

**Patent documents cited in the description**

- WO 2021102039 A
- US 11624088 B

**Non-patent literature cited in the description**

- Visium Spatial Gene Expression Solution. *Genomics*, 2019
- **RODRIQUES et al.** *Science*, 2019, vol. 363 (6434), 1463-1467
- **LIAO et al.** *bioRxiv*, 2023
- **GREENSTREET et al.** *bioRxiv*